(19)

Europäisches
Patentamt

European
Patent Office

Office européen
des brevets

(11) **EP 3 569 209 A1**

(12) **EUROPEAN PATENT APPLICATION**

(43) Date of publication:
**20.11.2019 Bulletin 2019/47**

(51) Int Cl.:
**A61F 13/537** (2006.01)

(21) Application number: **18172397.4**

(22) Date of filing: **15.05.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(71) Applicant: **The Procter & Gamble Company**
**Cincinnati, OH 45202 (US)**

(72) Inventors:
• **BIASUTTI, Cagda**
**65824 Schwalbach am Taunus (DE)**
• **KRIPPNER, Carola**
**65824 Schwalbach am Taunus (DE)**

• **MAZZEO, Michele**
**65824 Schwalbach am Taunus (DE)**
• **ROETTGER, Henning**
**24568 Kaltenkirchen (DE)**
• **EHMKE, Ralf**
**16945 Meyenburg (DE)**

(74) Representative: **P&G Patent Germany**
**Procter & Gamble Service GmbH**
**Sulzbacher Straße 40**
**65824 Schwalbach am Taunus (DE)**

(54) **DISPOSABLE ABSORBENT ARTICLES**

(57)    The present invention relates to absorbent hygiene articles, such as baby diapers, adult incontinence or feminine hygiene articles that incorporate an absorbent system. It is particularly suited for articles which are intended to receive more than one liquid gush load, as the absorbent system comprises an intermediate liquid storage member that holds the liquid of a gush and gradually releases it to an ultimate storage member exhibiting a smaller size than the intermediate storage member.

FIG. 1

EP 3 569 209 A1

**Description**

**Field of the invention**

The present invention relates to absorbent hygiene articles, such as baby diapers, adult incontinence or feminine hygiene articles and the like, incorporating an absorbent system. It is particularly suited for articles which are intended to receive more than one liquid gush load, as the absorbent system comprises an intermediate liquid storage member that holds the liquid of a gush and gradually releases it to an ultimate storage member, thus regenerating the intermediate liquid storage member for receiving the next liquid gush.

**Background**

In order for an absorbent article to provide low tendency for leakage as well as good skin condition of a wearer, the article may comprise an absorbent system, also referred to as absorbent core, that - among other requirements - quickly acquires the liquid and ultimately locks it away, also referred to as "storing" the liquid. Further, as the acquisition and the storage have different, and for an optimal performance often contradicting, liquid handling property requirements, absorbent systems further need to transfer or distribute the liquid from the acquisition region to the storage region. See e.g. US4699619, EP1075241 or EP1061878.

These publications also describe that the overall performance of absorbent systems can be improved by considering materials that are specialized for one of the functionality of acquisition, distribution and ultimate storage, respectively. Further the absorption-desorption properties of neighboring materials should be such that a first material readily receives the liquid and the ultimate storage materials firmly holds the liquid, once absorbed. Between acquisition and ultimate storage, the liquid is preferably easily spread longitudinally and/or laterally over the absorbent system by means of distribution materials, such as away from the loading region of the absorbent article in the crotch region of the wearer towards the front and rear end regions of the article positioned in the front and rear waist region of the wearer.

Structures exhibiting a high liquid transport capability are known from, e.g., US6506960 or US6727403, wherein low porosity membrane materials are described to unsheathe higher porosity core materials. Upon wetting of the membrane and the core materials, liquid can be very rapidly transferred from one region to another, optionally also against gravity (see, e.g., WO2000000138A1 or by a siphoning effect (see, e.g., EP1091714A2). However, this approach requires sophisticated and expensive membrane materials or a pre-wetting of the materials to initiate the liquid transport.

Yet a further approach describes an "intermediate storage layer" that can be designed to receive the liquid load faster than the ultimate storage material can, thus bridging the time between loading and ultimate storage, such as described in "Ultrathin Absorbent Solutions Enabling Design of Garment Like Disposable", H. Röttger, Insight Conference, Indianapolis, USA, 2014.

Further, increasing interest was raised and addressed with a multitude of approaches with regard to comfort and/or discreteness when wearing such articles. One approach, as also described in the EP1061878A1, aims at spatially separating the ultimate storage member from the loading region in the crotch region of the wearer, aiming at improving fit and putting additional performance challenges with regard to the ability of the materials to transfer the liquid from the loading point to the ultimate storage region.

However, there is still a need to provide articles that provide improved liquid handling performance at minimized material usage, but at the same time provide consumer benefits such as thinness, improved body fit or discreteness.

Henceforth, the present invention addresses one or more of these shortcomings and provide absorbent systems that are highly efficient with regard to material utilization whilst providing excellent liquid handling performance without compromising consumer benefits like softness, body fit or haptic properties.

**Summary**

The present invention relates to an absorbent hygiene article, exhibiting an x- or length direction, a y- or width direction and a z- or thickness direction, and comprising a liquid absorbent system comprising

A) an ultimate liquid storage region,
comprising an Ultimate Storage Member (USM) wherein the USM

- comprises
  A1) USM material exhibiting a basis absorbent retention of at least 2000 ml/m$^2$, whereby the USM material comprises Superabsorbent polymer (SAP) material;
- and exhibits
  A2) a dry thickness of less than about 3.0 mm, preferably less than about 1.0 mm, more preferably of less than about 0.8 mm, and

B) an intermediate storage region comprising an Intermediate Storage Member (ISM) comprising an ISM material, the ISM material exhibiting

B1) in the "Siphoning test"

B1a) - a Capillary Stall Height of more than about 45 mm, preferably of more than about 60 mm, even more preferably more than about 80 mm;

B2) - in the "Dynamic ISM Run-off Test"

B2) an Intermediate Storage Capacity of at least about 6.7 $g_{liq}/g_{mat}$, preferably of at least about 9.1 $g_{liq}/g_{mat}$, more preferably more than about 11 $g_{liq}/g_{mat}$.

In the liquid absorbent system

C1) the ultimate liquid storage region exhibits x-y-directionally an area of less than 85%, preferably less than 75% more preferably less than about 50% of the area of the intermediate storage region, and

C2) the USM exhibits a capacity of at least about 2500 $g_{liq}/m^2$, preferably at least about 3000 $g_{liq}/m^2$, more preferably at least about 4000 $g_{liq}/m^2$, when submitted to the "Combined ISM and USM Material Run-off Test upon application of a liquid load adapted to the basis weight of the overlying ISM.

In the liquid absorbent system, the USM material may further satisfy one or more of the conditions selected from the group consisting of

A3) exhibiting a flexural rigidity (x-directional) of less than about 35 mN*cm, preferably less than 20 mN*cm, more preferably less than 5mN*cm;

A4) exhibiting a Circular Bending of less than about 3.8 N, preferably less than about 2.7 N;

A5) exhibiting a compressibility of less than 9%;

A6) exhibiting an absorbent retention capacity of more than about 15 $g_{liq}/g_{mat}$, preferably more than 20 $g_{liq}/g_{mat}$, even more preferably more than 25 $g_{liq}/g_{mat}$ and most preferably of more than 30 $g_{liq}/g_{mat}$;

A7) exhibiting an absorbent retention Basis Capacity of at least about 25 $g_{liq}/m^2$, preferably at least about 28 $g_{liq}/m^2$, more preferably at least about 40 $g_{liq}/m^2$;

A8) an absorbent retention effective capacity of more than about 3 $g_{liq}/cm^3$.

The ISM material may further satisfy one or more of the conditions selected from the group consisting of

B1) exhibiting in the "Siphoning test"

B1b) - a Flow Rate of at least 1.7 $g_{liq}/min$, preferably more than about 2.8 $g_{liq}/min$, even more preferably of more than 4.1 $g_{liq}/min$

B1c) - a vertical wicking of more than 35 mm, preferably more than 65 mm, most preferably more than 90 mm;

B2) - in the "Dynamic ISM Run-off Test"

B2b) a run-off value of less than 50 %, preferably less than 20 % and most preferably of essentially 0 %;

B3) exhibiting in the "static ISM run-off test";

B3a) an absorption capacity of at least about 14 $g_{liq}/g_{mat}$, preferably of at least about 15 $g_{liq}/g_{mat}$, more preferably more than about 18 $g_{liq}/g_{mat}$;

B3b) a Run-off of less than about 40%, preferably less than about 20%, more preferably less than about 15%;

B4) exhibiting a flexural rigidity (MD) of less than 32 mN*cm, preferably less than 27 mN*cm, more preferably less than 5 mN*cm;

B5) exhibiting a Circular Bending of less than about 0.75 N, preferably less than about 0.50 N, more preferably less than 0.4 N;

B6) exhibiting a compressibility of more than about 14 %, preferably more than about 15 %, more preferably more than about 18%.

The combination of the ISM material and the USM material may further satisfy at least one of the requirements selected from the group consisting of

C3) exhibiting, when tested according to the 1st variant of the "Dynamic ISM/USM Liquid Distribution Test" with a liquid load of a fixed amount of test fluid of 50 g,

> C3a) a USM loading of at least about 40%, preferably more than about 54%, and even more preferably at least about 80% of the test fluid;
> C3b) a run-off of not more than about 60%, preferably less than about 30%, even more preferably less than about 10%, or even no detectable run-off at all;

C4) exhibiting, when tested according to the 2nd "variant of the "Dynamic ISM/USM Liquid Distribution Test", after loading with an amount of test fluid being adapted to the intermediate storage capacity of the ISM as determined according to the "Dynamic ISM run-off test",

> C4a) a USM loading of more than about 60 %, preferably more than 75 %, and even more preferably more than about 78 % of the liquid load;
> C4b) a Run-off of less than about 30%, preferably less than about 5 %, and most preferably essentially zero % run-off;

C5) exhibiting when tested according to the 3rd "variant of the "Dynamic ISM/USM Liquid Distribution Test"," after loading with an amount of test fluid being adapted to the basis weight of the ISM,

> C5a) a USM loading of more than about 60 %, preferably more than 75 %, and even more preferably more than about 78 % of the liquid load;
> C5b) a run-off of less than about 30%, preferably less than about 5 %, and most preferably essentially zero % run-off;

C6) exhibiting when tested according to the "Repeated ISM/USM Post Acquisition Rewet Test" exhibiting a rewet value of less than about 0.15 g, preferably less than about 0.05 g rewet;
C7) exhibiting when tested according to the "ISM recovery test at multiple loading"

> C7a) a difference of less than 30 %, preferably less than 10% between the 2nd and 3rd loading for
>
> > C7ai) the ISM load in % of load;
> > C7aii) the ISM load in $g_{liq}/g_{mat}$;
> > C7aiii) the ISM load in $g_{liq}/m^2$;
>
> C8) the flexural rigidity of the ISM being lower than the flexural rigidity of the USM;
> C9) the Circular Bending of the ISM being lower than the Circular bending of the USM.

Preferably, the ISM material comprises synthetic fibers, preferably bicomponent fibers, and cellulosic, preferably pulp, fibers, and a latex binder. Preferably, the ISM material is of the airlaid material type. Preferably, the USM material comprises SAP material at a concentration of at least about 80%, preferably at least about 90 % and more preferably of at least 95%, and further comprises cellulose, preferably pulp, and synthetic fibers. The USM material may further comprise latex and maybe of the airlaid material type.
The absorbent article may be positioned in the lower waist region of a wearer, comprising in Cartesian coordinates a length or longitudinal extension or x-direction, a width extension, and a thickness extension perpendicular to the length and the width.
The article comprises a front portion, for being positioned towards the front waist region of a wearer during use, a rear portion, for being positioned towards the rear waist region of a wearer during use, and a crotch portion positioned between the front and the rear portion. Further, a first surface is intended to be oriented towards a wearer during use, also referred to as topsheet surface and adapted to receive liquid bodily exudates of the wearer, preferably the surface of an article cover or topsheet. A z-directionally opposite surface is intended to be oriented outwardly and away from a wearer.
The article further comprises an absorbent system as described herein above positioned between the first and opposite surface of the article and aligned with the corresponding x-, y-, and z-direction of the article, such that the ISM region is positioned towards the first surface, and the USM region is positioned towards the opposite surface.
Such an absorbent article may be further selected from the group consisting of:

D1) a day use feminine hygiene article exhibiting an article retention capacity of more than about 2 ml but less than about 7 ml;
D2) a medium capacity hygiene pads exhibiting an article retention capacity of more than about 10 ml but less than about 50 ml;
D3) a high capacity incontinence article exhibiting an article retention capacity of more than about 50 ml, but less than about 300 ml;
D4) an ultra-high capacity incontinence article exhibiting an article retention capacity of more than about 300 ml,

wherein the article comprises

- a USM material exhibiting a retention capacity of more than about 10 $g_{liq}/g_{mat}$, preferably more than 15 $g_{liq}/g_{mat}$, more preferably more than 19 $g_{liq}/g_{mat}$, and
- an ISM exhibiting an ISM intermediate absorption capacity of at least about 5 $g_{liq}/g_{mat}$, preferably at least about 6 $g_{liq}/g_{mat}$, more preferably of at least about 9 $g_{liq}/g_{mat}$, and even more preferably of at least about 11 $g_{liq}/g_{mat}$.

## Brief description of the Figures

FIG. 1 is a perspective view of one example of an absorbent article that incorporates an absorbent system.
FIG. 2 is a representative cross-sectional views of the absorbent article of FIG. 1 taken through line 2-2.
FIGs. 3, 4A, 4B, 5A and 5B depict schematically test stands for evaluating materials and structures suitable for being employed according to the present invention.

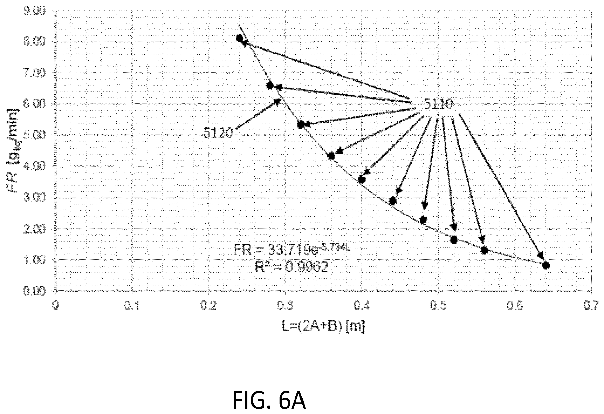

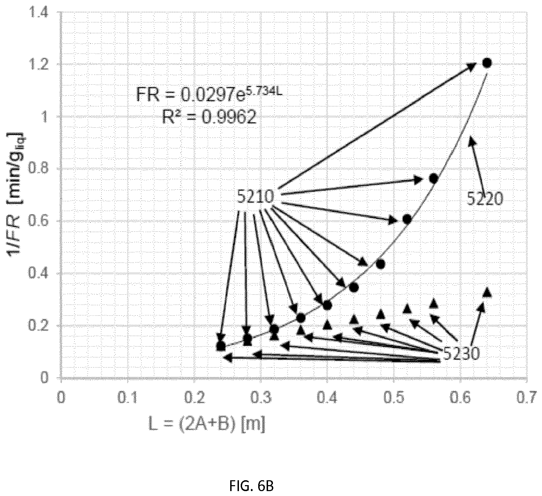

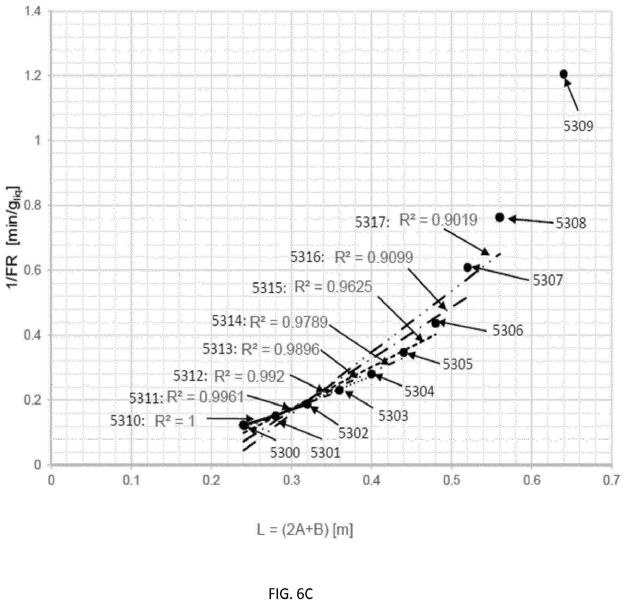

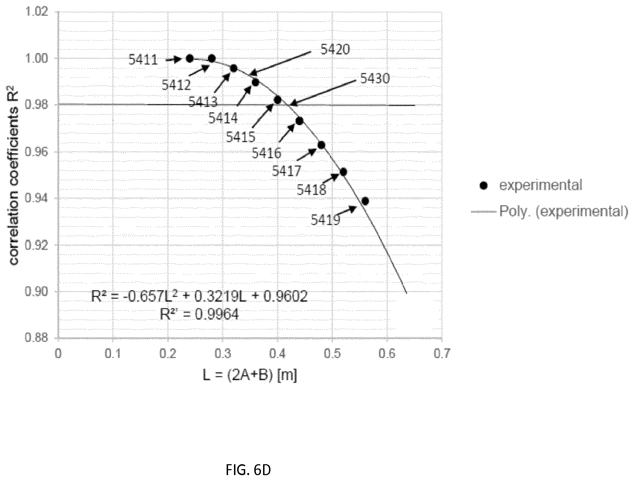

FIGs. 6A to D depict exemplarily diagrams useful for the evaluation of the Siphoning Test. While the specification concludes with claims particularly pointing out and distinctly claiming the subject matter that is regarded as the present invention, it is believed that the invention will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.

## Detailed description

The present disclosure relates to absorbent articles, such as disposable absorbent hygiene articles comprising an absorbent system.
Within the present context, absorbent hygiene articles are products to be worn, for example, in the lower crotch region of a wearer and intended to receive and retain bodily exudates, such as urine, faeces, menstrual fluids and the like, and intended for a single use and subsequent disposal in an environmentally and hygienically compatible manner. The present invention is particularly suitable in applications where multiple insults are occurring over the wearing period, which may last from a few hours in so-called day-usage conditions to ten hours or even longer, e.g. at overnight conditions. Thus, it is particularly well suited for baby diapers or training pants or for adult incontinence products, as may be diapers, or pants or incontinence or menstrual pads. The described absorbent system is also suitable for other fields of application such as but not limited to wound pads.
Such absorbent articles exhibit a liquid absorbency as may spread over a wide range depending on the intended use. Generally, when quantifying the liquid absorbency of an article, an absorbent system, an absorbent member, or a material, two measures are applied within the present context, namely the "retention capacity", as determined by centrifugation, also sometimes referred to as "centrifuge capacity", and the "absorbent drip capacity". Both determinations are executed by modifications of conventional test methods, as described in the Test Method section herein below. Further the capacities may be expressed by volume or by the weight of the absorbed liquid, though this difference is often numerically negligible. Absorbent capacities may be expressed by their volume in ml or weight in $g_{liq}$, obviously connected by the density of the liquid, which in the current context is the one of body liquids, typically and unless otherwise expressly noted, equated with the one of 0.9 % by weight of saline as synthetic urine. The values may be normalized by relating them to the weight of an absorbent system, an absorbent member, or a material, to be expressed as "$g_{liq}/g_{mat}$" or "$ml/g_{mat}$", or corresponding units. Within the present context, the volume of various members in the absorbent system is important, such that it may be advantageous to normalize the absorbent capacities of articles, absorbent systems, absorbent members, or materials to their volume, i.e. to their "retention capacity density" or "absorbent drip capacity density", expressed as "$g_{liq}/cm^3$" or $ml/cm^3$" or corresponding units. Within the present context, also the area extension of various articles, absorbent systems, absorbent members, or materials may be important, such that it may be advantageous to normalize the absorbent capacities of these to their area, i.e. to their "basis retention capacity" or "basis absorbent drip capacity" (in analogy to express weights of materials as "basis weights"), and expressed in "$g_{liq}/m^2$" or "$l/m^2$" or corresponding units.

The present disclosure maybe of particular benefit for various categories of articles, as may be categorized for the present context as follows by referring to an article retention capacity as the sum of the products of the retention capacities of the materials and their weights:

- Day use feminine hygiene pads intended to absorb low loads of menstrual liquid or urine, e.g., for slight involuntary urine losses ("drip incontinence"). Such articles may exhibit an article retention capacity of more than about 2 ml but typically less than about 7 ml.
- Medium capacity hygiene pads intended to absorb medium loads of menstrual liquid, such as in overnight use, or urine, e.g., for medium involuntary urine losses ("stress incontinence"). Such articles may exhibit an article retention capacity of more than about 10 ml but typically less than about 50 ml.
- High capacity incontinence articles, which may be in a diaper or pant form and at sizes to fit babies or adults, whereby the article may be worn around the waist of a wearer, or in a pad form, intended to absorb higher loads of urine, as may be occurring with urge or with overflow incontinence. Such articles may exhibit an article retention capacity of more than about 50 ml, but typically less than about 300 ml.
- Ultra high capacity incontinence articles, which are typically executed in diaper or pant form and at sizes to fit babies or adults, intended to use urine loads of more than about 300 ml.

In particular for higher absorbencies, it is well known and well established to use superabsorbent polymer ("SAP") material as an absorbent material.

One non-limiting embodiment of such an absorbent feminine hygiene pad or sanitary napkin will be specifically illustrated and described, although any features or elements of the feminine hygiene pad that are disclosed are also contemplated for any other embodiment of absorbent article, including incontinence pads.

Within the present context, an absorbent article - as well as its chassis or absorbent system elements - is considered in Cartesian coordinates to exhibit a length or longitudinal extension or x-direction, a width or y-directional extension, and a thickness or z-directional extension perpendicular to the length and the width - all seen also with respect to the positioning on a wearer such that the longitudinal extension of the article is aligned with a line extending from the front waist region of the wearer through a crotch region towards the rear waist region and the width or y- extension being aligned with the left-right orientation of a wearer. For the chassis and absorbent system, the respectively same orientation applies.

An absorbent article further comprises several portions, that are spatially separated, and that may further comprise sub-portions. Thus, the article comprises in a spatial or geometrical view

- a front portion, for being positioned in the front waist region of a wearer during use;
- a rear portion for being positioned in the rear waist region of a wearer during use;
- and a crotch portion positioned between the front and the rear region.

Especially the front and rear portions may, and often do, comprise sub-portions, such as laterally extending side panels.

Considering an absorbent article in a functional view, the article comprises several regions adapted to perform a primary function. Spatially such regions may be within one portion or sub-portion, or extend across two or more (sub-)portions.

A region comprises respective "region materials", that may be a unitary material, i.e. made up of essentially one type of material with essentially homogeneous composition and properties, or a composite material, made up of a mixture or spatial arrangement of unitary materials, that may form sub-regions. The spatial arrangement may be in essentially z-directionally spaced layers, or x-y-directionally spaced sub-regions. The one or more materials in a region may be homogeneous throughout the region or may exhibit a gradual change in composition and/or properties, such as density. Prior to use, the materials in the regions are essentially dry, which, however includes the usual residual moisture that materials may comprise, e.g., typically "dry" fluff pulp can comprise about 5 % moisture when stored at 23°C at 50% relative humidity. The "dry" materials as referred to in the present context are merely as in equilibrium with the surrounding atmosphere not considered to have undergone a particular drying step unless otherwise expressly noted (e.g. when additionally added moisture, such as from adding latex suspension, is removed again).

Thus, within the present context, an absorbent article comprises at least an acquisition region onto which the bodily exudates are deposited, i.e. which is during use in registry with the bodily openings like urethral or vaginal opening, penis, or anus. The acquisition region may just be a dedicated portion of the user oriented surface of the article, i.e. the topsheet, or may comprise particular acquisition materials, such as well-known open porous, often fibrous, webs.

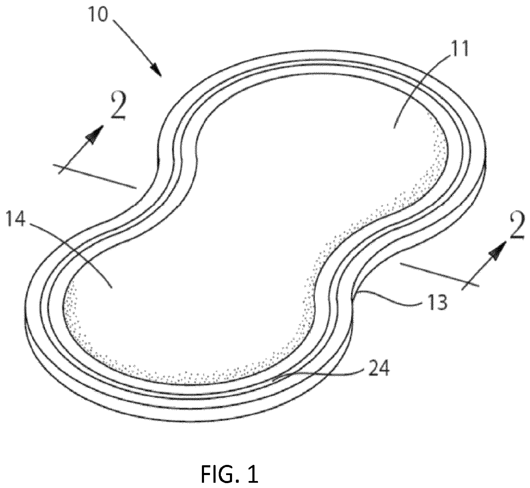

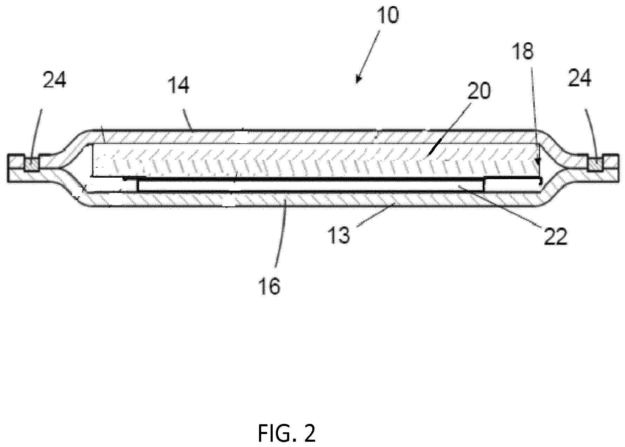

An absorbent article, for example a sanitary napkin 10 as shown in FIG. 1, can have any shape known in the art for feminine hygiene articles, including the generally symmetric "hourglass" shape shown in FIG. 1, as well as pear shapes, ovals, oblong ovals, droplet shapes, bicycle-seat shapes, trapezoidal shapes, or wedge shapes. Sanitary napkins and pantiliners can also be provided with lateral extensions known in the art as "flaps" or "wings" (not shown in FIG. 1). Such extensions can serve a number of purposes, including, but not limited to, protecting the wearer's panties from soiling and keeping the sanitary napkin secured in place. The illustrated absorbent article has a body-facing upper side that contacts the user's body during use. The opposite, garment-facing lower side contacts the user's clothing during use. The upper side of the sanitary napkin 10 generally has a topsheet 14 that can be liquid pervious. The lower side (seen in FIG. 2) has a backsheet 16 that is often liquid impervious and is joined with the topsheet 14 at the edges 12 of the sanitary napkin 10. The backsheet and the topsheet may be secured together in a variety of ways, for example with adhesive, heat bonding, pressure bonding, ultrasonic bonding, dynamic mechanical bonding, a crimp seal, or by any other suitable securing method. As shown in FIG. 2, a fluid impermeable crimp seal 24 can resist lateral migration ("wicking") of fluid through the edges of the product, inhibiting side soiling of the wearer's undergarments.

As is typical for sanitary napkins and the like, the sanitary napkin 10 of the present disclosure can have panty-fastening adhesive disposed on the garment-facing side of the backsheet 16. The panty-fastening adhesive can be any of known adhesives used in the art for this purpose, and can be covered prior to use by a release paper, as is well known in the art. If flaps or wings are present, a panty fastening adhesive can be applied to the garment facing side so as to contact and adhere to the underside of the wearer's panties.

A liquid storage system 18 is positioned between the topsheet 14 and the backsheet 16. The illustrated sanitary napkin 10 has a body facing upper side 11 that contacts the user's body during use. The opposite, garment-facing lower side 13 contacts the user's clothing during use. As shown in FIG. 2, the liquid storage system 18 may include a intermediate storage member (ISM) 20 for drawing liquid into the sanitary napkin from the topsheet and a ultimate storage member (USM) 22 where exudates are eventually held.

The topsheet 14 and the backsheet may be joined directly to each other in the periphery of the sanitary napkin or they maybe indirectly joined together by directly joining them to the absorbent core 18 or additional optional layers within the chassis, such as a secondary topsheet.

Topsheet

The absorbent article may comprise any known or otherwise effective topsheet, such as one which is compliant, soft feeling, and non-irritating to the wearer's skin. Suitable topsheet materials include a liquid pervious material that is oriented towards and contacts the body of the wearer permitting bodily discharges to rapidly penetrate through it without allowing fluid to flow back through the topsheet to the skin of the wearer. A suitable topsheet can be made of various materials such as woven and nonwoven materials; aperture film materials including aperture formed thermoplastic films, aperture plastic films, and fiber-entangled aperture films; hydro-formed thermoplastic films; porous foams; reticulated foams; reticulated thermoplastic films; thermoplastic scrims; or combinations thereof. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, meltblown, hydroentangled, combinations of the above, or the like. Suitable nonwoven materials may include low basis weight nonwovens, that is, nonwovens having a basis weight of from about 18 g/m2 to about 25 g/m2.

Topsheets may be formed by one or more of the layers made of the materials mentioned above, where one layer forms the outer surface of the absorbent article and one or more other layers are positioned immediately below it. The layer forming the outer surface of the article is typically a nonwoven layer or a formed film and it can be treated to be hydrophilic using surfactants or other means known to the person skilled in the art. Topsheets may additionally be aperture, have any suitable three-dimensional feature and/or have a plurality of embossments (e.g., a bond pattern). The topsheet may additionally be provided with tufts, formed with a laminated topsheet having an apertured upper layer and nonwoven lower layer, with "tufts" formed from the nonwoven layer protruding through the apertured upper layer.

Backsheet

The backsheet acts as a barrier to any absorbed bodily fluids that may pass through the absorbent core to the garment surface thereof with a resulting reduction in risk of staining undergarments or other clothing. Further, the barrier properties of the backsheet permit manual removal, if a wearer so desires, of the interlabial absorbent article with reduced risk of hand soiling. The backsheet may be positioned adjacent a garment-facing surface of the liquid storage system and may be joined thereto by attachment methods (not shown) such as those well known in the art. For example, the backsheet may be secured to the liquid storage system by a uniform continuous layer of adhesive, a patterned layer of adhesive, or an array of separate lines, spirals, or spots of adhesive. Alternatively, the attachment methods may comprise using heat bonds, pressure bonds, ultrasonic bonds, dynamic mechanical bonds, or any other suitable attachment methods or combinations of attachment methods. Forms of the present disclosure are also contemplated wherein the absorbent core is not joined to the backsheet, the topsheet or both.

The backsheet may be impervious, or substantially impervious, to liquids (e.g., urine) and may be manufactured from a thin plastic film, although other flexible liquid impervious materials may also be used. As used herein, the term "flexible" refers to materials which are compliant and will readily conform to the general shape and contours of the human body. The backsheet may prevent, or at least inhibit, the exudates absorbed and contained in the liquid storage system from wetting articles of clothing which contact the absorbent article such as undergarments. However, in some instances, the backsheet may permit vapors to escape from the liquid storage system (i.e., it is breathable) while in other instances the backsheet may not permit vapors to escape (i.e., non-breathable). Thus, the backsheet may comprise a polymeric film such as thermoplastic films of polyethylene or polypropylene. A suitable material for the backsheet is a thermoplastic film having a thickness of from about 0.012 mm to about 0.051 mm, for example. Any suitable backsheet known in the art maybe utilized with the present invention.

Another suitable backsheet material is a polyethylene film having a thickness of from about 0.012 mm to about 0.051 mm. The backsheet may be embossed and/or matte finished to provide a more clothlike appearance. For a stretchable but non-elastic breathable (i.e., permeable to water vapour and other gases) backsheet, a hydrophobic, stretchable, spun laced, non-woven material having a basis weight of from about 30 to 40 $g/m^2$, formed of polyethylene terephthalate or polypropylene fibers may be used. Other suitable breathable backsheets for use herein include single layer breathable backsheets which may be breathable and liquid impervious, and backsheets formed of two or more layers which in combination provide breathability and liquid imperviousness. For example, the backsheet may have a first layer comprising a gas permeable aperture formed film layer and a second layer comprising a breathable microporous film layer.

Where the backsheet is formed of a nonwoven web, it may have a basis weight of between 20 $g/m^2$ and 50 $g/m^2$.

Liquid Storage System

Within the present context, an absorbent article comprises a liquid storage system, which further comprises an ultimate storage member as may be abbreviated "USM", and an intermediate storage member as may be abbreviated "ISM". These members comprise materials that perform the function of the respective member, which are arranged within the respective regions.

In a preferred execution, the ultimate and the intermediate storage members are positioned z-directionally as x-y-directionally extending layered materials. These materials may exhibit a z-directional gradient of sub-layer structures.

It is a particular aspect of the present disclosure, that the USM and the ISM are in a particular spatial arrangement in that they are z-directionally super positioned and in liquid communication, preferably in direct liquid communication without any further material between them. The ISM covers x-y-directionally a larger area than the USM, which is fully covered by the ISM. Both members, and in particular the USM, are designed to be thin and soft so as to provide comfort and allowing positioning in the crotch portion of the article. In a further particular aspect, the ISM maybe even softer than the USM material such that when an article is worn between the legs of a wearer and the USM exhibits a smaller cross-directional extension than the ISM, as will be discussed more herein below, the even softer ISM material extends laterally outwardly towards the legs of a wearer.

It is a particular feature of the present disclosure, that in the liquid storage system the USM and ISM are particularly adapted to each other such that the USM provides the ultimate storage capability, whilst the ISM provides an intermediate storage functionality to receive and retain a gush load (ml or $g_{liq}$) delivered at a gush rate (i.e., ml/sec or $g_{liq}$/sec) and releasing the liquid to the USM at an ultimate storage rate which is a function of the USM materials allowing the use of USM materials with a low speed of acquisition. The USM has not only the capability to acquire and store liquid but also to draw or suck liquid from the ISM even from portions of the ISM that not directly overlay the USM, that is transported towards the zone overlaying the USM by capillary action. The thusly drained ISM can recover from the previous loading allowing re-loading of the ISM at a subsequent gush.

Accordingly, the ISM is adapted to retain the liquid not received by the USM to thusly prevent leakage, but also to release the liquid according to the suction capability of the USM.

It should be noted that in certain aspects of the present disclosure the USM and ISM are considered separate members, i.e., they are clearly discernible in an article, and may be added separately during the manufacturing of such an article. Surprisingly, it has been found that the intermediate storage and release property as an interplay between the ultimate storage member and the intermediate storage member has hitherto not been considered appropriately in corresponding design options. This is less surprising, when considering that no appropriate test method is known to establish appropriate parameter to allow appropriate design criteria. Thus, the pore structure of a suitable ISM should be well balanced so as to provide sufficient intermediate storage capacity whilst at the same time sufficient good liquid transport, also against gravity by small pores providing high capillary forces. This also supports the transfer of liquid from the ISM into the USM by pulling or sucking liquid out of the capillary system of the ISM into the USM. However, another aspect of the balancing relates to the suction of air into the capillary system, which "frees" up volume for liquid loading, but also can interrupt the liquid flow through the capillaries. While small pores help to prevent air getting pulled into the pore system when liquid gets sucked out, small pores also increase the flow resistance of the porous medium creating a larger pressure drop and reduced flow of liquid. We have found that it is desirable for the design of a suitable ISM/USM absorbent system to choose the right pore system providing sufficient capillary force for intermediate liquid storage and still sufficient liquid flow and easy transfer of liquid from the ISM to the USM

Aspects of the present disclosure are directed to a liquid absorbent system as maybe used in an absorbent core for an absorbent article of the various categories as described hereinabove. To this end, in one aspect of the present disclosure the required article absorbency is pre-determined so as to satisfy the absorbency requirements for the intended use. Then, both required capacity (e.g., retention capacity) and required or preferred size of a USM are determined and then this is combined in a particular arrangement with "a matching" ISM so as to provide an improved absorbent system allowing efficient use of materials at good performance, as maybe expressed in consumer benefits of dryness, non-leakage, softness, and wearing comfort both when dry and loaded.

Ultimate Liquid Storage Member (USM)

A first requirement for the USM is that it in a dry state it has to provide a certain liquid absorbency at a low thickness and good flexibility.

Considering intended use areas such as baby diapers, adult incontinence articles, or feminine hygiene articles, the article should be adapted to receive at least about 2 ml, or more than about 10 ml, or even more than about 300 ml, can be required as design capacity. The USM should be adapted to receive and retain such loads without overly contributing to the thickness of the article, such that the thickness of the unloaded dry USM is less than about 3 mm, often less than about 1 mm or even less than about 0.8 mm, preferably less than 0.5 mm or even less than about 0.3 mm.

The x-y-extending region for the USM should be such that it is comfortable to the wearer and typically exhibits

- a width of less than about 200 mm, preferably of less than about 140 mm, often less than about 100 mm or even less than about 80 mm, whereby for particular applications even widths of less than about 60 mm or even less than about 40 mm may be desirable;
- and a length of less than about 200 mm, preferably less than about 150 mm or even less than about 100 mm.

Thus, USM materials exhibit preferably an absorbent drip capacity for 0.9 % saline solutions of at least 20 $g_{liq}/g_{mat}$, more preferably more than 23 $g_{liq}/g_{mat}$ and even more preferably of more than 30 $g_{liq}/g_{mat}$ or a retention capacity of at least 15 $g_{liq}/g_{mat}$, more preferably more than 16 $g_{liq}/g_{mat}$ and even more preferably of more than 19 $g_{liq}/g_{mat}$. In order to enable thin product designs a high absorption density ($g_{liq}/cm^3$) is desired providing a high absorption capacity in a small volume, i.e. for 0.9 w-% saline solutions an absorption drip capacity density of at least 3 $g_{liq}/cm^3$ more preferably more than 6 $g_{liq}/cm^3$, and even more preferable more than 10.0 $g_{liq}/cm^3$ or even more than 11.0 $g_{liq}/cm^3$, or a retention density of more than about 1.0 $g_{liq}/cm^3$, preferably more than about 3.0 $g_{liq}/cm^3$, more preferable more than about 4.0 $g_{liq}/cm^3$ and even more preferable more than 5.0 $g_{liq}/cm^3$, and most preferable of more than about 7.0 $g_{liq}/cm^3$.

The Basis Absorption Drip Capacity of the USM may then be more than about 3.0 $l/m^2$, preferably more than about 6.0 $l/m^2$, more preferably more than about 8.0 l/m or more than about 10.0 $l/m^2$ or even more than 15.0 $l/m^2$. As an increase in basis capacity may lead to an increase in thickness of the loaded article and thus may negatively impact on the wearing comfort during use, the basis retention capacity should not exceed about 20.0 $l/m^2$, which corresponds to about 20.0 mm of liquid height (i.e. neglecting the volume of the absorbent material) and often less than about 10.0 $l/m^2$ are desirable. The Absorption drip capacity density of the USM may then be more than about 3.0 $g_{liq}/cm^3$, preferably more than about 6.0 $g_{liq}/cm^3$, more preferably more than about 7.0 $g_{liq}/cm^3$ or even more than about 10.0 $g_{liq}/cm^3$. In order to achieve high liquid absorbency in a relatively thin structure, relatively high SAP content is desired for the USM. Thus, the SAP content of the USM is typically well above about 60 %, often more than about 75 %, desirably more than about 85 %, and preferably more than about 90 %, more preferably more than about 97 % or even more than about 99 %, all on a weight basis of the USM material. In the extreme, the USM may be made of pure SAP material, i.e. exhibiting an SAP concentration of 100 %. If the USM material exhibits an uneven SAP concentration across its x-y extension, the SAP concentration should be determined and averaged for an area of ate least 10 mm by 10 mm. Thus, the SAP concentration may often range from about 60 % to about 99 %, or from about 70 % to about 98 % or from 85 % to about 95 %. However, in contrast to conventional designs, for which for high SAP material concentrations, especially for pure SAP material layers, detrimental gel-blocking occurs, in aspects of the present disclosure the relatively low thickness of the USM, combined with the unique arrangement of ISM and USM, can significantly alleviate or even eliminate the negative effects of gel-blocking.

For good comfort for a user, USM materials should exhibit a good flexibility, such as by exhibiting a Flexural Rigidity (as referred to in the Test Method section herein below) that is preferably less than 100 mN*cm, preferably less than 50 mN*cm, more preferably less than 20 mN*cm or by exhibiting a circular bending (as referred to in the Test Method section herein below) that is preferably less than 10 N, preferably less than 5 N and more preferably less than 3 N. It should be noted USM materials should provide a soft feel as a result of the good flexibility. However, if this requirement is satisfied, a USM material does not necessarily need to exhibit a good "cushiness" or "compressibility softness", but even essentially incompressible materials, like an SAP film or highly compressed SAP particle layer, may very suitably be employed. The basis weight of SAP material is from about 35 $g/m^2$ to about 100 $g/m^2$ or even about 150 $g/m^2$ or even more than about 300 $g/m^2$, still whilst exhibiting a thickness of less than about 3 mm, often less than about 1 mm or even less than about 0.8 mm, preferably less than 0.5 mm or even less than about 0.3 mm.

The USM comprises materials that exhibit high liquid absorbency, as, e.g., described in the above referenced EP10611878, to which express reference is made for the "Materials to achieve Storage Absorbent Member requirements". Soft and flexible materials that may be suitable for the present invention may also comprise SAP fibers, with such materials being commercially available under the designation SAF® as from Technical Absorbents, UK, and may be incorporated into airlaid or carded webs, preferably at high SAF ® concentration and high compression.

Further, for a suitable USM material comprising SAP particles these may be laminated between liquid permeable layers, such as conventional paper tissue layers, e.g., at basis weights of 18 $g/m^2$, or hydrophilic nonwoven materials, such as conventionally used for topsheets in absorbent articles. Such materials are commercially available, such as from Gelok International, OH, US, under the trade designation Gelok® laminate, or from Domtar, SC, US, under the trade designation NovaZorb®.

The USM contains absorbent material, such as creped cellulose wadding, fluffed cellulose fibers, Rayon fibers, wood pulp fibers also known as airfelt, and textile fibers. The USM further includes superabsorbent material that imbibes fluids and forms hydrogel. In an embodiment, the USM comprises a first and second cellulose layer with super absorbent materials disposed therebetween. In this case, the USM may be laminated with mechanical compression (rather than with use of adhesives). Such super absorbent materials may be included in particle form. SAP is typically capable of absorbing large quantities of body fluids and retaining them under moderate pressures. Synthetic fibers including cellulose acetate, polyvinyl fluoride, polyvinylidene chloride, acrylics (such as ORLON), polyvinyl acetate, non-soluble polyvinyl alcohol, polyethylene, polypropylene, polyamides (such as nylon), polyesters, bicomponent fibers, tricomponent fibers, mixtures thereof and the like can also be used in the USM. The USM may also include filler materials, such as PERLITE, diatomaceous earth, VERMICULITE, or other suitable materials that lower rewet problems.

The USM may have SAP in a uniform distribution or in a non-uniform distribution, for example, in the form of channels, pockets, stripes, criss-cross patters, swirls, dots, or any other pattern, either two or three dimensional known arrangement.

Particularly suitable materials for the USM herein have been described in EP2872097, and are commercially available from Glatfelter Falkenhagen, Germany, under the trade designation "eCore™", e.g. eCore™ 100, or eCore™ 270, or eCore™ 400. Such materials comprise an airlaid mixture of cellulose fibers and SAP particles, encased by surface layers of latex sprayed onto the cellulose, creating a highly absorbent yet thin, flexible and non-dusting structure.

Whilst suitable USM materials can be made in-line during the manufacturing process for absorbent articles with high production speeds of more than 300 m/min or even more than 500 m/min, it is often preferred, e.g., for process simplicity, that the USM materials are provided in a web or sheet form, such that they can be provided pre-formed to a converting unit for making the absorbent articles. In a preferred execution USM materials are roll-stock materials, i.e., maybe supplied in the form of a web, e.g., essentially continuous from a roll or spool, or from a box, thusly significantly reducing the complexity of the manufacturing process by eliminating a complex core forming process step, or by reducing dust generation, in particular when the materials comprising pulp also comprise binder fibers and surface applied latex dispersion binder.

It is a particular benefit of the present invention that the so called "gel-blocking" phenomenon known from structures with high amounts of SAP material is significantly alleviated or eliminated. Without wishing to be bound by theory, this is currently believed to primarily be due to the low thickness of the USM that allows sufficient z-directional liquid transport by osmotic transport mechanisms. This is in contrast to the multitude of prior art approaches that aim at enabling or enhancing liquid transport through the USM, such as by increasing the permeability of the USM or - in particular for USM with high concentrations of SAP material - of the SAP material itself, such as expressed by the saline flow conductivity parameter. Thus, even USM materials that would exhibit a relatively high tendency to undesirable gel blocking in other systems may be used according to the present disclosure, as the liquid distribution within the teachings herein is taking place in the ISM driven by capillary force on top of the USM, i.e., the liquid transfer within the USM does not primarily rely on capillary liquid flow between the SAP particles and/or the fibers but instead primarily on the relative slow diffusion and gel swelling mechanisms to provide sufficient suction force to drain liquid out of the ISM. Because of the relative slow diffusion and gel swelling process it is preferable that the ISM on top of the USM has the capability to intermediately store the liquid prior to the liquid finally getting stored in the USM.

Intermediate Storage Member (ISM)

The ISM cooperates with the USM in that it receives and holds the liquid as delivered to the absorbent system until the USM has absorbed it by its relatively slower absorption mechanisms as described in the above.

To this end, for functioning well in the present context, the ISM should exhibit balanced properties of liquid storage and retention, but also of transport and release of the liquid, thusly exhibiting a balance of liquid wicking or capillary suction capability and flow resistance of liquid flowing through the materials.

Surprisingly this balance required for an efficient removal of liquid from the ISM into the USM can be well established by determining the capillary stall height and flow rate according to the Siphoning Test, described in detail in the Test Method Section. The measurement principle is based on general liquid flow correlations adapted and simplified to match the current conditions, especially

the Hagen-Poiseuille equation

$$(Eq\text{-}1) \qquad Q = (r^4 * \pi * delta\,(p))/\,(8 * \eta * L),$$

wherein Q stands for the volumetric flow rate (in $m^3$/sec). r for the radius of a capillary (in m), L for the length of the capillary (in m), $\eta$ for the dynamic viscosity of the liquid (in Pa*sec), delta (p) for the pressure differential (in Pa), and $\pi$ the circle constant.

and the suction pressure

$$(Eq\text{-}2) \qquad delta\,(p) = g * \varrho * B,$$

wherein delta (p) stands for the pressure differential (in Pa), $\varrho$ for the density of the liquid (in kg/$m^3$), B for the suction height differential (in m), and g for the gravity constant (in m/$s^2$).

The capillary stall height is describing the capability to drain liquid out of the ISM by suction describing the capability to pull liquid across the structure by sufficient capillary force avoiding a disruption of the liquid within the capillary system by pulling air into the capillary system when the pressure drop inside the capillary created by the applied suction is larger than the capillary force of the pore system (measured via the wicking height) discontinuing the flow of liquid in the capillary system driven by the applied suction. In contrast to conventional vertical or inclined wicking tests, the Siphoning test takes into consideration the pressure drop created in the system under dynamic conditions similar to in-use when liquid is pulled out of the ISM by the USM. Smaller pores provide higher capillary forces and higher stall height favourable for the capability to remove liquid from the ISM. By application of suction force, such as from a high suction power absorbent material, especially from suction by osmotic swelling of a SAP material, the capillary forces of the pore system can be overcome, however the smaller pores also create a larger pressure drop or flow resistance in the system, requiring a higher suction power to achieve higher flow rate. Smaller pore size results in an increased flow resistance detrimental to higher liquid transport rates within the ISM, both when liquid is loaded onto the material, but also when it is removed by the USM. A well-functioning ISM provides a suitable balance between a sufficiently high capillary suction (described by the stall height) and a sufficiently low flow resistance not limiting the flow of liquid into and out of the ISM.

Conventionally, these aspects are seen to be contradicting, as in porous systems good liquid storage and retention is connected to a smaller pore size, whilst good liquid transport implies low liquid flow friction and hence larger pore sizes. Thus, conventional systems often employ "acquisition materials" that are very open and as such allow quick acquisition as well as little flow resistance for gravity driven flow, but neither have a good interim storage capacity nor good, if any, wicking capability against gravity. Hence such acquisition materials are typically combined with separate "distribution" materials, that can accomplish the interim storage function and - at least to a certain degree - also some wicking against gravity.

Thus, when being submitted to the Siphoning Test, suitable ISM materials exhibit a Capillary Stall Height of more than about 35 mm, preferably of more than about 60 mm, more preferably more than about 80 mm, and even more preferably of more than about 85 mm.

Suitable materials exhibit a flow rate FR (at A = 20 mm and B = 200 mm) of at least about 3.0 $g_{liq}$/min, preferably of at least about 6.0 $g_{liq}$/min, more preferably of more than about 8.0 $g_{liq}$/min, preferably even more than 14.0 $g_{liq}$/min.

Suitable ISM materials may exhibit a specific flow rate of 1.5 ($g_{liq}$/min) /·$g_{mat}$, preferably more than 2.0 ($g_{liq}$/min) /·$g_{mat}$, more preferably more than 4.0 ($g_{liq}$/min) /·$g_{mat}$.

Suitable materials may also be described by their matter constant K, as also resulting from the Siphoning test and described herein below.

Further, it is preferred for suitable ISM materials to exhibit rapid liquid intake, distribution and intermediate storage. These properties may be evaluated by the "Dynamic ISM Material run-off test", as described in more detail herein below, assessing the ability of an ISM material, to receive, retain and release a test liquid, which is deposited under gravity driven flow conditions onto the material at an inclined position.

Thus, a suitable ISM material should rapidly take in and distribute the liquid whilst having sufficient capillary force to hold the liquid in the pore structure even if the samples is positioned at a slope and not positioned horizontally. Suitable ISM materials show a minimal run-off value. Run off leakage can result from too slow liquid intake such that liquid flows on top of the material and dripping off before it can penetrate into the pore structure. Run off leakage can also occur when large pores allow rapid intake of the liquid but only provide limited capillary force to hold the liquid in the porous structure. A suitable ISM material provides a pore structure that is beneficial by providing sufficiently fast liquid intake and a sufficiently high capillary force to hold the liquid in the porous structure.

Thus, a suitable ISM material can be selected when it exhibits

- a residual liquid load in the ISM material - also referred to as Intermediate Storage Capacity of the ISM - of at least about 5.0 $g_{liq}/g_{mat}$, preferably of at least about 9.0 $g_{liq}/g_{mat}$, more preferably more than about 11.0 $g_{liq}/g_{mat}$; and / or
- a Run-off of less than about 50%, preferably less than about 20%,

when tested according to the Dynamic ISM Run-off Test.

Further, useful ISM materials preferably exhibit balanced liquid release properties for releasing the liquid to the USM, and preferably only to the USM so as to avoid undesired leakage. This can be determined in the "Static ISM run-off test", as described in more detail herein below. This test describes the capability of the ISM material to store and hold liquid against gravity in the porous structure by capillary force. A large total pore volume and small pore size are beneficial for a good static intermediate storage layer which, however, needs to be balanced with the liquid intake capability as described in the context of the Dynamic ISM Run-off test previously described. Thus, the specimen is soaked in the test liquid and subsequently transferred to a horizontal steel grid, where the liquid can drip out from the wet specimen for a pre-set time. The wet specimen is transferred from the flat configuration to an inclined test stand (as for the Dynamic ISM Run-Off Test), upon which only a small amount of run-off should occur and more of the liquid should remain in the ISM material.

Thus, a suitable material can be selected when it preferably exhibits

- a static ISM load of at least about 14.0 $g_{liq}/g_{mat}$ preferably of at least about 15.0 $g_{liq}/g_{mat}$, more preferably more than about 18.0 $g_{liq}/g_{mat}$; and / or
- a Run-off of less than about 40%, preferably less than about 20%, more preferably less than about 15%,

when tested according to the Static ISM run-off test.

For good comfort as well as skin health for a user, ISM materials should exhibit a good flexibility and softness. Whilst these properties may be perceived differently by different user, suitable ISM materials should preferably exhibit a stiffness, as may be determined by the Flexural Rigidity test (as referred to in the Test Method section herein below) of less than about 100 mN*cm, preferably less than about 50 mN*cm, more preferably less than about 20 mN*cm. Additionally or alternatively, the materials may exhibit a low bending resistance, such as by exhibiting a Circular Bending Value (as referred to in the Test Method section herein below) of preferably less than 1.00 N, more preferably less than 0.75 N and even more preferably less than 0.50 N. Yet another aspect of user relevant mechanical properties relates to "cushiness" as maybe described by the compressibility (as referred in the Test Method section herein below), which should be more than 8 %, preferably more than 14 % or even more preferably more than 20 %.

Examples of fibers suitable for use in the ISM include synthetic or regenerated fibers selected from PET, polyethylene, polypropylene, nylon, rayon, polylactic acid, multicomponent binder fibers and mixtures thereof.

In addition to the materials described above, the ISM can comprise a wide variety of liquid-absorbent materials commonly used in disposable absorbent articles. Non-limiting examples of liquid-absorbent materials suitable for use include comminuted wood pulp which is generally referred to as airfelt or pulp; creped cellulose wadding; chemically stiffened, modified, or cross-linked cellulose fibers, cotton fibers; meltblown polymers including co-form; synthetic fibers including crimped polyester fibers; capillary channel fibers; absorbent foams; absorbent sponges; synthetic staple fibers and superabsorbent polymers (SAP).

The ISM may be formed as a unitary structure - meaning that although it may be formed by several layers that have distinct properties and/or compositions from one another, they are somehow intermixed at the boundary region so that, instead of a definite boundary between layers, it would be possible to identify a region where the different layers transition one into the other. Such a unitary structure may be built forming the various sub-layers one on top of the other in a continuous manner, for example using air laid or wet laid deposition. Typically, there is no adhesive used between the sub-layers of the unitary material. However, in some cases, adhesives and/or binders can be present although typically in a lower amount that in multilayer materials formed by separate layers.

In an embodiment, the ISM may have a fibrous nonwoven layer comprising fibers having an average length from 26 to 200 mm. In some embodiments, the average fiber size in dtex can be selected so as to be in the range of from 0.5 to 15 dtex. The average fiber length is measuring according to ASTM method D5103-07 and the average size in dtex according to the ASTM method D1577-07. The nonwoven layer that may form the unitary ISM can have a basis weight of from 10 to 100 gsm and a thickness from 0.2 to 5 mm and can be selected from needlepunched, hydroentangled, air through bonded, spunbonded, carded resin bonded, and melt blown nonwoven materials. (specific to the NW carrier layer). Air through bonded carded nonwovens are in some cases preferred because this consolidation technology can result in materials having a good z-direction compression resistance, and good capillarity even at low basis weight (thus allowing to manufacture thinner and lower cost absorbent elements).

The nonwoven layer of the ISM can be manufactured from an assortment of suitable fiber types that produce the desired mechanical performance and fluid handling performance. In some embodiments, an air through bonded carded nonwoven may be formed from a combination of stiffening fibers. The stiffening fibers, for example, can form about 20% to about 40%, by weight, of the air through bonded carded fiber nonwoven. In other embodiments, the stiffening fibers can form about 100%, by weight, of the nonwoven.

The stiffening fibers can be polyethylene terephthalate (PET) fibers, or other suitable noncellulosic fibers known in the art. The PET fibers can have any suitable structure or shape. For example, the PET fibers can be round or have other shapes, such as spiral, scalloped oval, trilobal, scalloped ribbon, and so forth. Further, the PET fibers can be solid, hollow or multi-hollow. In some embodiments of the carded fiber nonwoven, the stiffening fibers may be fibers made of hollow/spiral PET. Other suitable examples of stiffening fibers include polyester/co-extruded polyester fibers. The stiffening fibers may be multicomponent binder fibers, where individual fibers are provided from different materials, usually a first and a second polymeric material. The two materials may be chemically different (hence the fibers are chemically heterogeneous) or they may differ only in their physical properties while being chemically identical (hence the fibers are chemically homogeneous). The stiffening fibers may also be a blend of multicomponent fibers with polyester fibers.

With specific reference to multicomponent fibers comprised of a polypropylene/polyethylene fiber composition, in a cross-sectional view of a fiber, the material with a higher softening temperature can provide the central part (i.e, the core) of the fiber. The core typically is responsible for the bicomponent fiber's ability to transmit forces and have a certain rigidity or otherwise provide structures with resiliency. The outer coating on the core (i.e., the sheath) of the fiber can have a lower melting point and is used to facilitate thermally bonding of substrates comprising such fibers. In one embodiment, a polypropylene core is provided with a polyethylene coating on the outside, such that about 50%, by weight, of the fiber material is polypropylene and 50%, by weight, of the fiber material is polyethylene. Other quantitative amounts can of course be selected. For example, bicomponent fibers can have a composition from about 30% to about 70%, by weight, polyethylene, while others have about 35% to about 65%, by weight polyethylene. In some embodiments, bicomponent fibers can have a composition from about 40% to about 60% or about 45% to about 55%, by weight, polyethylene.

Unitary structures in absorbent elements for absorbent articles are known in the art and described for example in are described in in WO03/090656A1 from Procter & Gamble, US2002/007169A1 from Weyerhaeuser and WO00/74620A1 from Buckeye. These documents describe liquid storage systems having a unitary structure.

Alternatively, the unitary structure can be obtained by forming the ISM as an airlaid material where the at least two sublayers forming it are deposited in subsequent steps on a single airlaid line directly onto the wire carrier and then latex applied to the body facing and garment facing surfaces to ensure proper binding and/or reduce dustiness of the material.

In both embodiments, the sub-layers are formed on an air laid machinery having several forming heads (in general one for each sub layer although it could be imagined that one forming head could form two or more non adjacent layers or that two forming heads could deposit the same composition, thereby forming a single sub-layer) and wherein each forming head lays down a specific combination of materials in a given set of conditions. In this process a first forming head forms a first air laid layer, then a second forming head forms a second air laid layer on top of the first layer. The process goes on until the desired series of sub-layers is obtained. Typically during the deposition of an air laid layer or sub-layer the composition of the materials (e.g., % of multi-component fibers) deposited by each forming head is constant, however it is possible to envision embodiments where the composition of the materials of each forming head varies. This allows generating a continuous variation of composition and properties of the material along its z axis in a single layer or sub-layer. In the case where more forming heads are present it is possible to conduct compression steps between the passage from one forming head to another.

When the deposition of the air laid ISM is complete the resulting material may be compressed to compact it (e.g., via calendaring). In case multicomponent binder fibers are present, the material can be thermally treated at a temperature above the softening temperature of a bonding component of the multicomponent binder fibers and below the softening point of a structural component in the multicomponent binder fibers so that the binder fibers can bind among sub-layers. The ISM may additionally be embossed which may be beneficial for the wet integrity of the ISM and to increase its density. The resulting sheet of material can then be cut if necessary in the appropriate size and used as absorbent element within the absorbent core of an absorbent article or combined with an acquisition layer to form an absorbent element.

Interaction of ISM and USM

It is preferable that, as depicted in FIGs. 2, 2B and 2C, the USM 22 exhibits an area that is smaller than the area of the ISM 20, here shown by exhibiting a y-directional extension smaller than the ISM and preferably also an x-directional extension that is smaller x-directional extension as compared to the ISM 20. Typically, and as shown, though not necessarily, both the USM and the ISM are positioned symmetrically and centered to a longitudinal centerline. Typically, and as shown, though not necessarily, the USM is positioned in the article such that it fits into the crotch region of a wearer, but it does not need to be positioned longitudinally symmetrically the crotch point of the wearer, nor to the longitudinal center point of the article, but it can be shifted for- or rearwardly, as schematically indicated in Fig. 1. Further, at least a portion of it should be positioned within the crotch portion of the article, and may, but does not need to, correspond to the crotch portion. For some product designs, it even may be favorable to have a USM with two or more sub-elements the ISM to guide liquid into certain zones for ultimate storage, improve the recovery of the ISM at minimal material consumption of UMS or to design textile properties like flexible zones etc. favorable to improved body fit of the absorbent article. For example, USM patches may be placed longitudinally offset, and / or laterally offset, e.g. in a stripe design.

It is a particular benefit of the present invention that it allows to design absorbent systems providing good comfort and fit to a wearer by designing the USM to a substantially smaller size than the ISM while providing a good liquid management. This allows to position the USM unobtrusively, e.g., in the crotch region, whilst allowing the larger ISM to capture the exudates over a larger area.

Thus, the USM extends over a region that x-y-directionally corresponds to less than 90%, preferably less than 70%, more preferably less than 50% of the area of the ISM region.

Such a smaller area maybe achieved by the USM exhibiting a length extension that is less than 90%, preferably less than 70%, more preferably less than 50% of the longitudinal extension of the ISM, and/or a width extension that is less than 100%, preferably less than 90%,or even less than 50% of the width of the ISM.

Ultimately, the liquid exudates shall be stored in the USM without being released back to the ISM or even to the skin of the wearer during normal in use conditions. The ISM receives the liquid, transports it both x-y- and z-directionally driven by capillary flow, and intermediately stores the liquid by capillary force along its larger x-y-extension ISM material that hitherto was defined by the loading area onto which the gush of liquid is released. The ISM then releases the liquid to the USM in an interplay with the suction properties of the USM, thusly providing a dry user-oriented surface. The release of the liquid to the USM regenerates the ISM and allows repeating of the cycle until exhaustion of the capacity of the USM. The design of the capillary system of the ISM provides sufficient capillary force to drive the liquid from the top-sheet facing surface towards the USM facing surface and distributing the liquid according to the required storage volume but also allowing the movement of the liquid from the ISM to the USM driven by the suction provided by the USM whilst emptying the pore-system of the ISM.

In the period between loading of the ISM and release of the liquid to the USM, the ISM needs to hold the liquid sufficiently strong by capillary force to avoid leakage and minimize rewet to the skin of the wearer - optionally supported by further layers, especially open pore acquisition layers, or topsheet materials.

Whilst the high capillary force of the small pores of the ISM is desired to hold the liquid therein during the intermediate storage period, the capillary force of the ISM needs to be sufficiently smaller than the suction force of the USM driving the flow of liquid from the ISM into the USM. The design of the absorbent system preferably assures a direct liquid contact between ISM and USM so that the high suction components of the USM directly interface the ISM and the liquid and can be removed from the ISM by a suction force substantially higher than the capillary force of the ISM. Superabsorbent Polymers are particularly suitable as the force of the osmotic swelling can be more than 100 times higher than the capillary force of typical ISM materials. As the initiation of osmotic swelling is often not immediate and may start after 30 sec or even longer after wetting, and as the liquid intake by osmotic swelling is much slower that typical liquid release into hygiene articles, it is preferable that the ISM is able to rapidly take in and intermediately store the liquid until the USM can absorb the liquid.

The interaction of the ISM and the USM can be very advantageously be assessed by combining ISM materials and USM materials in under well defined conditions - especially regarding their respective size and choice of materials. Once a well matching pair of materials has been determined, a skilled person will be readily capable of adjusting specific parameters such as basis weights or SAP material type and content to the specific requirements for the specific in-use application, based on the teachings herein. It certain aspects it is further important that the absorbency properties of the ISM and USM materials, in particular the high storage force or high capillary pressure of a small pore size and the desired low flow resistance of a larger pore size, are adapted to each other. To this end, the "Dynamic ISM/USM Liquid Distribution Test", as described herein below in the test method section, allows assessing the interaction of the ISM and the USM materials by evaluating the partitioning or distribution of liquid within a combination of ISM and USM materials by positioning an USM material of certain size on an inclined surface overlaid by a larger ISM material. After applying the test fluid to the surface of the ISM, the amount thereof in the ISM material, in the USM material as well the run-off from both materials is determined describing the capability of a combined ISM and USM system to take in the test liquid, distribute and intermediately store the liquid in the ISM and then release the liquid to the USM, thusly emptying and regenerating the ISM for the next gush of liquid. Performing the test with the sample positioned with a slope reflects in-use conditions where the absorbent core is not always positioned horizontally or the absorbent core of the hygiene article is exposed to some pressure. It is desired that the run-off leakage of liquid is minimal and that the majority of the liquid is transferred into the USM showing the capability of the system to regenerate the ISM for the next gush of liquid and to move the majority of the liquid into the USM where it gets ultimately stored and held by the osmotic forces of the swelling SAP material, thus not being released again. This should be even the case, if the structure is exposed to a high pressure as may occur during use (e.g., more than about 100 kPa) at which liquid remaining in the ISM and only held by capillary force can get squeezed out by application of moderate pressure, even of less than about 10 kPa, corresponding to the wicking height of the ISM.

The more of the liquid is transferred into the USM material at a minimized run-off, the better the system components ISM und USM are adapted to each other. Pairs of ISM and USM materials may be assessed by variants of the test, as described in more detail in the test method section herein below.

In a first variant, the materials may be submitted to a constant liquid load of 50 g per gush, allowing to compare a situation that may be close to real use of the absorbent core of an article. For this test variant, a suitable USM material should absorb at least about 40 %, preferably more than about 50 %, and more preferably more than about 60 %, and most preferably at least about 80 % of the test fluid, and exhibit at a run-off of not more than about 60 %, preferably less than about 30 %, even more preferably less than about 10 %, or even more preferably no detectable run-off at all. Accordingly, the resulting liquid absorbency of the USM material on a unit area preferably exceed about 2000 $g_{liq}/m^2$, more preferably is more than about 3000 $g_{liq}/m^2$, or even more preferably more than about 4000 $g_{liq}/m^2$.

However, this test alone might not necessarily reflect efficient use of the material, as just changing to a higher basis weigh of the ISM may result in better performance, albeit at increased cost. In order to compare ISM materials of different basis weight a second variant of the test is favorable, adjusting the liquid load according to the basis weight of the ISM material. Then suitable material combinations should result in low run off, preferably less than 25 %, more preferably less than 15 %, even more preferably less than about 5 % and most preferably essentially zero run-off. Preferably at least about 60 %, more preferably more than about 70 % and even more preferably more than about 80 % of the liquid load is transferred into the USM material.

ISM materials exhibiting the same basis weights may show different intermediate liquid storage performance, whichmaybe reflected by a third variant of the test wherein the liquid load is adjusted according to the intermediate liquid storage capacity of the ISM material as determined in the "Dynamic ISM run-off Test". Then suitable material combinations should result in low run off, preferably less than 25 %, more preferably less than 5 % and most preferably essentially zero run-off. Preferably at least about 60 %, more preferably more than about 75 % of the liquid load is transferred into the USM material demonstrating a good capability of the ISM to get regenerated by transferring liquid to the USM.

Overall, thin USM materials with a very high SAP content show good performance when in combination with a suitable ISM as selected according to the teaching described herein, demonstrating that the system of a larger sized ISM combined with a well-matched but smaller sized USM overcomes performance limitation otherwise caused by gel-blocking, as the liquid distribution takes place in the ISM while the liquid only or at least predominantly needs to penetrate the USM in a vertical direction, which is beneficial for very thin USM with high SAP material content allowing the design of very thin absorbent systems.

When an ISM / USM combination is loaded, the liquid distributes between the ISM and USM. Once the USM is given sufficient time to suck the liquid out of the ISM, a suitable ISM should have recovered, and be ready for a further gush. However, the suction power of the USM may be reduced with an increased load. Hence in order to eliminate this effect, a patch of ISM material can be loaded multiple times, whilst the respective USM material with which it is combined, is refreshed after each loading and after a certain waiting time, as described in detail in Test Method Section herein below for the "ISM recovery Test". The liquid handling performance of the ISM should then preferably not deteriorate at all, at least when comparing the second and third gush, i.e. after the initial effects of the first gush are set.

Yet a further property of an absorbent system is the capability of quickly acquiring a liquid load and retaining it under an applied pressure, especially not allowing the liquid to reach wearer's skin ("rewet"). Under low load loading and sufficient waiting time between subsequent gushes (see "Repeated ISM/USM-system Post Acquisition Rewet Test" as described herein below), a suitable system exhibits an acquisition time for the first gush of less than about 5 sec, preferably less than about 3 sec, and more preferably less than about 1 sec. The acquisition time for the third gush is preferably less than about 15 sec, more preferably less than about 11 sec. The rewet value after the third gush is preferably less than about 0.16 g, more preferably less than about 0.10 g.

Yet another aspect of the present disclosure relates to the interplay of the ISM and USM with regard to the textile properties, as may be described by the flexural rigidity and circular bending tests as described herein below, wherein ISM materials and USM materials may exhibit different results. ISM materials preferably provide good flexibility, drapeability, and compressibility in z direction (which is also referred to as cushioness), whilst USM materials, and especially the SAP material containing USM materials, may exhibit a somewhat higher rigidity and lower compressibility in z-direction or even essentially no compressibility. When assessing these properties by the mentioned test methods, it should be noted that for evaluating the impact of the flexural rigidity on the perceived softness and flexibility of a material, the mass and density of the material need to be considered. Materials with a high density have a low flexural rigidity parameter due to the higher mass pushing the sample down in the test set-up. Therefore, it is important to also look at parameters like the circular bending to get a better estimate for the flexibility and drape properties of a material. Thus, the concept of combining a wider ISM on top of a smaller USM allows to take advantage of the favourable textile properties of the ISM while moving the less textile like USM into a smaller zone of the hygiene product allowing the design of very comfortable hygiene products. Especially in the crotch region it is very beneficial having a narrow USM for reducing the risk of chafing the skin of the wear, or even not getting in touch with the legs of the user, whilst the more textile-like ISM is perceived as soft and flexible when getting in touch with body parts of the consumer.

Manufacturing of absorbent systems and absorbent articles

The manufacturing of absorbent articles is well known in the art, typically comprising the combining of various materials, many of which are prefabricated and often provided as web materials or "roll stock goods", on a manufacturing line. In the present context, the introduction of the USM and ISM to form an absorbent system is preferably executed such that the materials forming these members are provided separately as an essentially continuous webs or cut pieces from such a web, more preferably being unwound from rolls or spools or provided from festooned blocks, to form clearly discernible layers. However, such materials may also be formed in-line, such as when the USM is made by positioning SAP particles onto an ISM in a web-form and covering it by a cover material web or by positioning SAP particles on a carrier and positioning the ISM on top of the SAP particle layer. Preferably, though not necessarily, the webs from which the ISM or USM are formed, exhibit a uniform thickness.

Thus, the making of structures according to the present invention may employ, for example

- providing materials, such as roll stock materials from unwinds, bulk materials like SAP particles, optionally adhesive materials;
- size adapting and positioning these relative to each other such as by cut-and-space technology or zoned material deposition for bulk materials like SAP particles or adhesives;
- connecting respective materials as necessary;
- inserting such materials into conventional converter for the manufacturing of absorbent articles.

Exemplary Materials

Various suitable USM and ISM materials have been selected and evaluated for their properties as such or in combination.

USM-1 is a material commercially available from Glatfelter Falkenhagen, Germany, under the trade designation eCore™ VH400.104. It has been made according to the teachings of EP2872097 on a Neumag OERLIKON airlaid line and comprises multiple layers of pulp and SAP particles, a tissue, and a surface applied binder spray.

USM-2 is a material commercially available from Glatfelter Falkenhagen, Germany, under the trade designation eCore™ VH270.203. It has been made according to the teachings of EP2872097 on a Neumag OERLIKON airlaid line and comprises multiple layers of pulp and SAP particles, a tissue, and a surface applied binder spray.

USM-3 is a material commercially available from Glatfelter Falkenhagen, Germany, under the trade designation MH460.101. It is a multi-bonded multiple layer airlaid material comprising pulp, SAP particles, bicomponent fibers and latex dispersion binder.

USM-4 is a material of Glatfelter Falkenhagen, Germany, under the designation eCore+ - CS200/111 and comprising 200 g/m$^2$ of particulate SAP EK-X EN52 from supplier Ekotec sandwiched between two layers of wetlaid tissue material of 18 g/m$^2$ each. The laminate is highly compressed by two calender rolls to a thickness of 0.3 mm, thereby achieving high integrity with good flexibility but reduced cushioness. Similar tissue/SAP/tissue laminates are produced by Domtar according to the patent WO 2011/084981 A1 or Gelok International (e.g. 5040-72 S/S).

USM-5 is a material commercially available from McAirlaid's Vliesstoffe, Germany, under the trade designation SuperCore® Type T-502.

USM-6 is a material commercially available from Technical Absorbents, UK, under the trade designation Airlaid Fabric, type 2717, and comprising a SAF® (superabsorbent fiber), fluff pulp and Bi-Co fiber core, and a nonwoven top and bottom layer.

USM-7 is made from three layers of Airlaid Fabric, type 2351, commercially available from Technical Absorbents, UK, comprising superabsorbent fibers (SAF®), fluff pulp and Bi-Co fiber core, and a nonwoven top layer.

Further, several ISM materials have been tested and evaluated.

ISM-1 is commercially available from Glatfelter Falkenhagen, Germany, under the product code MH090.118 and made on a Neumag Oerlikon airlaid line as a multi-bonded airlaid material.
ISM-2 is commercially available from Glatfelter Falkenhagen, Germany, a under the product code MH095.110 and made on a Neumag Oerlikon airlaid line as a multi-bonded airlaid material.
ISM-3 is commercially available from Glatfelter Falkenhagen, Germany, under the product code MH120.129 and made on a Neumag Oerlikon airlaid line as a multi-bonded airlaid material.
ISM-4 is commercially available as Sawasoft 55 from Sandler GmbH, Germany, and is a hydro-entangled non-woven with polyester / bicomponent fibers and a basis weight of 55 g/m$^2$.
ISM-5 is commercially available as Airten 1160W from Berry, Italy, and is an air-through bonded non-woven (highloft) with polyethylene / polypropylene (50 %/50 %) bicomponent fibers and-a basis weight of 60 g/m$^2$.

Test results

The materials and exemplary combinations thereof, which should not be seen limiting in any way, were submitted to the tests as described in the test methods section. Table 1a and Table 1b summarize certain results for USM and ISM materials, respectively.

Table 1a - Summary of Properties of USM Materials:

| | | USM -1 | USM -2 | USM -3 | USM -4 | USM -5 | USM -6 | USM -7(*) |
|---|---|---|---|---|---|---|---|---|
| Basis weight | $g_{mat}/m^2$ | 400 | 270 | 460 | 288 | 525 | 240 | 76 |
| SAP content | % | 83 | 75 | 60 | 85 | 57. | n.a. | n.a. |
| SAP basis weight | $g_{mat}/m^2$ | 332 | 203 | 276 | 200 | 300 | n.a. | n.a. |
| Absorption Drip capacity (30 min) | $g_{liq}/g_{mat}$ | 30 | 25.5 | 23.7 | 30.1 | 24.7 | 16.2 | 11.4 |
| Absorption drip basis capacity | $kg_{liq}/m^2$ | 12.0 | 6.9 | 10.9 | 8.7 | 12.9 | 3.9 | 0.9 |
| Absorption drip density | $g_{liq}/cm^3$ | 11.8 | 7.7 | 6.1 | 10.1 | 6.6 | 3.0 | 1.1 |
| Absorption Retention capacity (30 min) | $g_{liq}/g_{mat}$ | 19.70 | 16.88 | 15.66 | 16.81 | 11.73 | 5.33 | 2.05 |
| Retention basis capacity | $kg_{liq}/m^2$ | 7.9 | 4.6 | 7.2 | 4.8 | 6.2 | 1.3 | 0.2 |
| Absorption Retention capacity density | $g_{liq}/cm^3$ | 7.9 | 4.6 | 7.2 | 4.8 | 6.2 | 1.3 | 0.2 |
| Stiffness as Flexural rigidity | mN*cm | 33.0 | 57 | 241 | 21 | 93 | 361 | 14 |
| Circular bending | N | 2.7 | 3.3 | 3.8 | 0.5 | 10.0 | 2.6 | 0.1 |
| Compressibility | % | 6.9 | 9.4 | 7.6 | 5.0 | 4.6 | 11.5 | 17.6 |
| Thickness | mm | 1.02 | 0.89 | 1.80 | 0.86 | 1.95 | 1.30 | 0.80 |
| Density | $g_{mat}/cm^3$ | 0.392 | 0.303 | 0.256 | 0.335 | 0.269 | 0.185 | 0.095 |
| (*) values for one layer | | | | | | | | |

Table 1b Summary of properties for ISM materials

|  |  | ISM-1 | ISM-2 | ISM-3 | ISM-4 | ISM-5 |
|---|---|---|---|---|---|---|
| Basis weight | $g_{mat}/m^2$ | 90 | 95 | 120 | 55 | 60 |
| Thickness @ 2.0 kPa | mm | 1.80 | 1.40 | 1.90 | 0.57 | 0.46 |
| Liquid Strike through | sec | 0.59 | 0.43 | 0.18 | 1.49 | 1.16 |
| Vertical wicking | mm | 36 | 68 | 67 | 90 | 10 |

However, it has been found that for particularly relevant properties of materials and material combinations suitable for the present disclosure, established test methods for determining appropriate parameter ranges were missing and thus particularly useful test methods have been developed for selecting suitable ISM and USM materials as well as combinations thereof. These test methods and the results are discussed in more detail herein below.

"ISM material - Siphoning Test"

A good ISM material requires a good balance between stall height and flow rate demonstrating the capability to drain the ISM even against gravity in case the material is not positioned horizontally and vertical wicking height demonstrating the capability to intermediately store liquid even against a certain pressure. As can be seen in "Table 2 - Siphoning Test", ISM-2 and 3, provide the most suitable materials with a stall height larger than 80 mm, a specific flow rate larger than 1.7 $(g_{liq}/min) / \cdot g_{mat}$ and a vertical wicking height of more than 65 mm. ISM-1 is considered acceptable showing a lower stall height of 63 mm but a higher flow rate of 4.1 $(g_{liq}/min) / \cdot g_{mat}$ and a vertical wicking height of 36 mm.
ISM-4 is considered as less suitable material with providing a good stall height of 84 mm but only a low specific flow rate of just 1.3 $(g_{liq}/min) / \cdot g_{mat}$ and a vertical wicking height of 90 mm. ISM-5 provides a too low stall height of just 37 mm but a good specific flow rate of 6.8 $(g_{liq}/min) / \cdot g_{mat}$ and a too low vertical wicking of just 10 mm indicating a poor capability of holding, i.e. intermediately storing, liquid.

Table 2 - ISM Material - Siphoning Test

|  | ISM | -1 | -2 | -3 | -4 | -5 |
|---|---|---|---|---|---|---|
| Capillary Stall height | mm | 63 | 89 | 81 | 84 | 37 |
| Flow rate @ A= 20 mm | $g_{liq}/min$ | 14.6 | 6.6 | 8.5 | 3.0 | 6.8 |
| Specific flow rate @ at A=20 / B= 200 mm | $(g_{liq}/min)/\cdot g_{mat}$ | 4.1 | 1.7 | 1.8 | 1.3 | 2.8 |
| Matter constant K | $(g_{liq}/min)$ | 53 | 22 | 31 | 9 | 24 |

"Dynamic ISM Run off Test"

Referring to the results as shown in Table 3, this test shows the capability of the ISM to take in and intermediately store the liquid revealing that ISM-3 exhibits the best results, whilst ISM-1, and -2 still provide good results, and samples ISM-4 and ISM-5 have poor or even very poor performance because of not being capable to hold the liquid. Specifically ISM-4 reveals a too slow intake of liquid resulting in liquid running off on the surface of the ISM while ISM-5 has a fast liquid intake but insufficient capillary force to hold the liquid against gravity force.

Table 3 - Dynamic ISM Run off Test

|  |  | ISM-1 | ISM-2 | ISM-3 | ISM-4 | ISM-5 |
|---|---|---|---|---|---|---|
| Intermediate Storage Capacity | $g_{liq}/g_{mat}$ | 11.0 | 9.1 | 11.7 | 6,7 | 5,8 |
| Liquid kept in ISM | % | 54 | 50 | 80 | 20 | 12 |
| run-off | % | 46 | 50 | 20 | 80 | 88 |

Static ISM Run-off Test

Different to the Dynamic Run-off Test showing the impact of speed of liquid intake (run-off on the surface) and capability to hold the liquid by capillary force, the Static Run-off Test excludes the effect of speed of liquid intake revealing the full effect of the capability of the ISM to hold the liquid by capillary force. Referring to the results as shown in Table 4, this test shows that samples ISM-1, -2, 3 and -4 have the best run-off performance being able to hold the liquid against the force of gravity while sample ISM-5 performs poorly only able to hold less than 75% of the liquid. Sample ISM-3 exhibits the highest ISM load value which is very beneficial, whilst sample ISM-4 has the lowest result for this parameter making it less suitable for use as ISM due to a lack of intermediate storage capacity in spite of the good capability to hold liquid shown by the low run-off of just 16%.

Table 4 - Static ISM Run-off Test

|  |  | ISM-1 | ISM-2 | ISM-3 | ISM-4 | ISM-5 |
|---|---|---|---|---|---|---|
| ISM load | $g_{liq}/g_{mat}$ | 15.2 | 14.0 | 18.1 | 10.7 | 17.2 |
|  | % | 80 | 85 | 79 | 84 | 75 |
| run-off | % | 20 | 15 | 21 | 16 | 25 |

Textile properties

The textile properties characterized by the flexural rigidity, circular bending and compressibility tested according to the methods as described herein below, demonstrate that materials suitable for ISM and USM in the present invention, can have - and preferably do have - significantly different mechanical properties. While ISM materials should exhibit good flexibility and especially compressibility in z direction as a measure for cushiness, especially USM materials, and even more especially the ones comprising high concentrations of particulate SAP, e.g.,. USM-1, USM-2, USM-3, USM-4, USM-5, may exhibit higher rigidity (in the specific test as herein referred to) and a lower compressibility in z-direction. When comparing the impact of the flexural rigidity on the softness respectively flexibility of a material as perceived by a consumer with the test results, the mass and density of the material need to be considered among other factors. Materials with a high density exhibit low values in the flexural rigidity test due to the higher mass that is pushing the sample down in the test set-up. Therefore, it is important to also look at results of the circular bending test for better assessing the flexibility, softness or drape of a material.
The concept of combining a larger and especially a wider ISM on top of a smaller USM allows to take advantage of more favourable textile properties of the ISM material whilst positioning the USM material, which shows a less textile-like behaviour, into a smaller zone of the hygiene product allowing the design of very comfortable hygiene products. Especially in the crotch area of the article on a wearer, it is very beneficial having a narrow USM that is shielded from the skin of the wearer, such as of the legs, by the softer, more textile-like ISM which is perceived as being soft and flexible, both when being applied to or by a user and during wear-time.

Table 5 - Textile property parameters of ISM materials

|  |  | ISM-1 | ISM-2 | ISM-3 | ISM-4 | ISM-5 |
|---|---|---|---|---|---|---|
| Flexural rigidity | mN*cm | 31.5 | 26.3 | 98.0 | 1.62 | 6.29 |
| Circular bending | N | 0.50 | 0.40 | 0.75 | 0.03 | 0.03 |
| Compressibility (between 2.0 kPa and 0.5 kPa) | % | 15 | 14 | 18 | 16 | 31 |

Dynamic ISM/USM Liquid Distribution Test

This test simulates the in-use situation considering the capability of the ISM/USM system to rapidly take in liquid and intermediately store it without leakage by run-off before efficiently transferring the liquid to the USM for final storage.
In its first variant, the test is executed with a constant liquid load of 50 g for all samples not considering the different basis weight of the ISM.
Referring to Table 6, various ISM materials are compared by being all tested with the same USM material, here USM-1. In this test, the ISM-3 showed the best performance (no leakage and 80% of liquid moved to the USM) and ISM-1, and -2 good or respectively acceptable (ISM-4) performance (moderate leakage < 32% and more than 50% of the liquid transferred to the USM), whilst sample ISM-5 shows poor performance (more than 50% leakage and 36% or less of the liquid transferred to the USM.

Table 6 - Dynamic ISM/USM Liquid Distribution Test - constant liquid load of 50 ml - various ISM materials

| all vs. USM-1 | | ISM-1 | ISM-2 | ISM-3 | ISM-4 | ISM-5 |
|---|---|---|---|---|---|---|
| load in ISM | % | 15 | 14 | 20 | 5 | 6 |
| load in USM | % | 60 | 56 | 80 | 36 | 22 |
| | $g_{liq}/m^2$ | 3000 | 2800 | 4000 | 1800 | 1100 |
| run-off | % | 25 | 31 | 0 | 50 | 72 |

Referring to Table 7, various USM materials are compared by being tested with the same ISM material, here ISM-3, and thusly compares the capability of a USM material to pull liquid out of the ISM under the dynamic test conditions. It is preferable, that good contact is created between the USM and the ISM for liquid transfer to enable sufficient suction by osmotic swelling of the SAP. As can be seen in Table 7, USM-3 showed the best results, followed by USM-1, -4, and -5. USM-2 and USM-6 appear still acceptable, whilst USM-7 performed poorest, which might be contributed to the fact that a sufficiently high absorbent capacity was reached by combining three layers of low basis weight and capacity material and the quite open porous structure of this material potentially not providing sufficient suction force to drain liquid out of the ISM.

Table 7 - Dynamic ISM/USM Liquid Distribution Test - constant liquid load of 50 ml - various USM materials

| All vs. ISM-3 | USM- | -1 | -2 | -3 | -4 | -5 | -6 | -7 |
|---|---|---|---|---|---|---|---|---|
| SAP content (see Table 1a) | % | 83 | 75 | 60 | 85 | 57 | n.a. | n.a. |
| load in ISM | % | 20 | 46 | 14 | 21 | 17 | 54 | 72 |
| | $g_{liq}/g_{mat}$ | 2.8 | 6.4 | 1.9 | 2.9 | 2.4 | 7.5 | 10.0 |
| | $g_{liq}/m^2$ | 333 | 767 | 233 | 350 | 283 | 900 | 1200 |
| load in USM | % | 80 | 54 | 86 | 79 | 83 | 46 | 28 |
| | $g_{liq}/g_{mat}$ | 3.3 | 2.3 | 3.6 | 3.3 | 3.5 | 1.9 | 1.2 |
| | $g_{liq}/m^2$ | 400 0 | 2700 | 4300 | 3950 | 4150 | 2300 | 1400 |
| run-off @ 10 mins | % | 0 | 0 | 0 | 0 | 0 | 0 | 0 |

In the second variant of Dynamic ISM/USM Liquid Distribution Test, the liquid load has been adapted to the basis weight of the various ISM materials, so as to assess the capability of the system to move the liquid from the ISM to the USM (smaller in size) positioned on a slope. Also in this test variant (see Table 8), the ISM-3 showed the best performance and ISM-1, and 2 good or acceptable performance, whilst samples ISM-4, and -5 showed poor performance, partly due to poor wetting properties such that surface run-off occurred.

Table 8 - Dynamic ISM/USM Liquid Distribution Test - liquid load adapted to ISM basis weight - various ISM material

| | | ISM-1 | ISM-2 | ISM-3 | ISM-4 | ISM-5 |
|---|---|---|---|---|---|---|
| Basis weight (see Table 1b) | $g_{mat}/m^2$ | 90 | 95 | 120 | 55 | 60 |
| liquid load =f{basis weight} | $g_{liq}$ | 38 | 40 | 50 | 23 | 25 |
| load in ISM | % | 18 | 21 | 20 | 6 | 10 |
| load in USM | % | 70 | 66 | 80 | 48 | 37 |
| | $g_{liq}/m^2$ | 2660 | 2640 | 4000 | 1104 | 925 |
| run-off | % | 12 | 13 | 0 | 46 | 53 |

In the third variant of the Dynamic ISM/USM Liquid Distribution Test, various ISM materials are compared against a single USM material, here USM-1 whilst the amount of liquid applied has been adjusted to the intermediate storage capacity of the specific ISM materials tested according to the Dynamic ISM Material Run-off Test, in order to measure the capability of the system to move the liquid from the ISM to the USM (smaller in size) even against a gravimetric force. Also in this test (see Table 9), the ISM-3 showed the best performance (zero leakage and absorbing the largest amount of liquid) and ISM-1, and 2 good or acceptable performance, whilst samples ISM-4, and -5 showed poor performance.

Table 9 - Dynamic ISM/USM Liquid Distribution Test - liquid load adapted to ISM capacity - various ISM materials using USM-1

| All vs. USM-1 | ISM- | -1 | -2 | -3 | -4 | -5 |
|---|---|---|---|---|---|---|
| Intermediate Storage Capacity (see Table 3) | | 11.0 | 9.1 | 11.7 | 6.7 | 5.8 |
| liquid load as function of Intermediate Storage Capacity | $g_{liq}$ | 25 | 25 | 40 | 10 | 10 |
| load in ISM | % | 19 | 23 | 22 | 10 | 9 |
| load in USM | % | 79 | 76 | 78 | 61 | 51 |
| | $g_{liq}/m^2$ | 1975 | 1900 | 3120 | 610 | 510 |
| run-off | % | 2 | 2 | 0 | 29 | 41 |

ISM recovery Test

In order to demonstrate the capability of regenerating the ISM by moving liquid into the USM, the "ISM recovery Test" has been executed as described herein below with patches of ISM-3 overlying USM-1 patches as being refreshed for each load, as described herein before with constant liquid load at 50 ml for each gush.

The data compiled in table 10 show the capability to recover the ISM (300 $cm^2$) using a USM (100 $cm^2$), i.e. only covering 1/3 of the surface of the ISM. Although there is a drop in performance (increased run-off and higher residual amount of liquid in the ISM) after application of the first gush of liquid which is expected to be related to structural changes of the ISM by interaction with the liquid, the performance of the ISM/USM assembly is constant over the 2nd and 3rd gush demonstrating the capability of the small USM to repeatedly remove liquid from the 3 times larger ISM.

Table 10 "ISM Recovery Test" - repeated run-off w / fresh cores

| | | 1st gush | 2nd gush | 3rd gush |
|---|---|---|---|---|
| Load in ISM-3 | % | 23 | 16 | 16 |
| | $g_{liq}/g_{mat}$ | 3.2 | 2.2 | 2.2 |
| Load in USM-1 | % | 74 | 55 | 55 |
| | $g_{liq}/m^2$ | 3700 | 2750 | 2750 |
| Run off | % | 3 | 30 | 29 |

In addition to a good Run-off performance, the system preferably provides a good skin dryness performance, as may be evaluated by the rewet, as may be tested on the ISM material after being removed from the USM according to the test method described herein below. This resulted for the combination of ISM-3 and USM-1 as tested according to the "ISM recovery Test" in a rewet value of 6.94 $g_{liq}$.

"Repeated ISM/USM-system Post-Acquisition Rewet Test"

In order to assess the acquisition time and rewet in a horizontal position of different ISM materials at a size of 300 mm x 100 mm as described, have been combined with a reference USM material, here a USM-1 material, remaining in the test for three intakes, at a size of 100mm x 100mm with the USM patch centred under the ISM patch at the application point of the liquid. The "Repeated ISM/USM system Post-Acquisition Rewet Test" provided the results as summarized in Table 11 and reveal good rewet data for all samples while the intake time is increasing from gush to gush showing that the ISM cannot be fully regenerated during the 10 minutes waiting period. The increase of intake time is largest for ISM-4 indicating a potential change of the pore structure (collapsing) after liquid application. Overall all ISM materials show a good performance.

Table 11 "Repeated ISM/USM system Post-Acquisition Rewet Test"

|  |  | ISM-1/ USM-1 | ISM-2/ USM-1 | ISM-3/ USM-1 | ISM-4/ USM-1 | ISM-5/ USM-1 |
|---|---|---|---|---|---|---|
| Fist intake | sec | 1.8 | 1.8 | 0.9 | 5.0 | 3.0 |
| Second intake | sec | 8.4 | 18.0 | 7.3 | 15.3 | 7.4 |
| Third intake | sec | 11.3 | 16.6 | 11.0 | 17.9 | 9.4 |
| Rewet | $g_{liq}$ | 0.16 | 0.15 | 0.09 | 0.10 | 0.06 |

## Test methods

### (Adapted) Standard Procedures

Unless expressly stated, for standard parameter test methods as have been developed together by EDANA and INDA, presently as the NWSP Nonwovens Standard Procedures 2015 edition, should be applied:
Generally, test methods should be executed under standard laboratory conditions of 23°C $\pm$ 2°C and 50% $\pm$ 5% relative humidity (see NWSP 003.0.R0 (15)). Samples should be conditioned under such conditions for 24 h prior to testing. Typical applied units in brackets.

Basis weight: NWSP 130.1.R0 (15) $[g_{mat}/m^2]$;
Thickness: NWSP 120.6.R0 (15) at 2.0 kPa [mm];
Stiffness of NW NWSP 090.5.R0 (15)
   using the bending length providing Flexural rigidity per unit width [mN*cm];
Compressibility NWSP 120.3 R0 (15)
   thickness at 0.5 kPa versus 2 kPa [%];
Vertical Wicking Test NWSP 010.1.R0 (15) para. 8.3
   liquid wicking rate (capillarity), with 5 min measuring time [mm];
Liquid Strike through NWSP 070.3.R0 (15) [sec];
Rewet after repeated liquid strike through: NWSP 070.8.R0 (15)
   modified by 10 minutes waiting time between gushes,
   results being acquisition time [sec] for 1st, 2nd, and third gush, [sec];
   rewet after last gush $[g_{liq}]$

The Circular bending Test is conducted on a mechanical property testing machine Zwick Z.2.5/TN1S, as is typically used to measure tearing strength of nonwovens according to NWSP 100.3.R0 (15). Additional special equipment is required to measure circular bending. The equipment consists of an upper cylindrical stainless steel piston with a total length of 72 mm and a diameter of 6 mm, and on the top a needle point with a length of 0.9 mm and a diameter of <0.5 mm. The piston is fixed on the upper arm of the Zwick device and drives towards a steel plate with the dimension of 102 x 102 x 6.4 mm, which has a hole with a diameter of 18.8 mm at the center point of the steel plate. The nonwoven test specimen of a size of 40 mm x 40 mm is placed over the hole of the steel plate. The steel plate is installed on a pedestal, which is fixed on the ground plate of the Zwick device. The testing machine measures the force, which the piston requires to move/bend the nonwoven material 6.35 mm through the hole of the steel plate, which is then reported in Newton N as result.

### Liquid absorbency

As to liquid absorbency of USM materials, NWSP methods have been used or adapted as follows:

- Absorption Drip Capacity for USM materials: Following the SAP Powder Free Swell determination NWSP 240.0.R2 (15), modifying it by setting the sample size to 100 mm x 50 mm to obtain a SAP content of approximately 1.3 $\pm$ 0.4 g in the teabag.
- Retention Capacity for USM materials: based on NWSP 241.0.R2 (15) ("Polyacrylate superabsorbent powders - Determination of the fluid retention capacity in saline solution by gravimetric measurement following centrifugation" - sometimes also referred to as teabag or centrifuge capacity) wherein the sample size is set to 100 x 50 mm to obtain a SAP content of approximately 1.3 ± 0.4 g in the teabag.

- Article / Composite / Member Retention Capacity is a useful measure for determining absorbency of a complete absorbent article of particular components thereof, such as of an absorbent core in an absorbent article. Such a retention capacity can be determined by submitting extracted portions of the article or material to the same testing conditions according to the "Retention Capacity for USM materials" (see above). Such an article or composite retention capacity can also be approximated by adding the retention capacities of the major absorbent compounds, mainly of the superabsorbent polymer materials and fibrous structures, as may be determined separately. For mixtures of conventional fluff pulp with SAP particles , a retention capacity of about 4 $g_{liq}/g_{mat}$ can be assumed for the pulp.

- Further the capacities may be expressed the volume or by the weight of the absorbed liquid, though this difference is often numerically negligible. The values may be normalized by relating them to the weight of an absorbent system, an absorbent member, or a material, to be expressed as "$g_{liq}/g_{mat}$" or "$ml/g_{mat}$", or corresponding units. When reporting absorbency values, care should be taken to distinguish the weight unit of the material, as referred to as [$g_{mat}$] to the weight of the liquid [$g_{liq}$]. Within the present context, the volume of various members an absorbent system is important, such that it maybe advantageous to normalize the absorbent capacities of articles, absorbent systems, absorbent members, or a materials to their volume, i.e. to their "retention capacity density" or "absorbent drip capacity density", expressed as "$g_{liq}/cm^3$" or "$ml/cm^3$" or corresponding units. Within the present context, also the area extension of various articles, absorbent systems, absorbent members, or materials may be important, such that it may be advantageous to normalize the absorbent capacities of these to their area, i.e. to their "basis retention capacity" or "basis absorbent drip capacity" (in analogy to express weights of materials as "basis weights"), and expressed in "$g_{liq}/m^2$" or "$l/m^2$" or corresponding units.

Siphoning test- refer to Fig. 2.

The test aims at describing the capability to pull liquid out of a porous system by application of a suction as it takes place when the USM sucks liquid out of the ISM. In determining the balancing of liquid wicking, resp. capillary flow and flow resistance by determining the capillary suction capability and the flow of the liquid through materials considered for use as ISM, expressed in capillary stall height and flow rate. For deducing relevant measurement parameters, general liquid flow correlations, especially - as already indicated in the above -
the Hagen-Poiseuille equation

$$(Eq\text{-}1) \qquad Q = (r^4 * \pi * delta\,(p))/\,(8 * \eta * L),$$

wherein Q stands for the volumetric flow rate (in $m^3/sec$) r for the radius of a capillary (in m), L for the length of the capillary (in m), $\eta$ for the dynamic viscosity of the liquid (in Pa*sec), delta (p) for the pressure differential (in Pa), and $\pi$ the circle constant.
and the suction pressure

$$(Eq\text{-}2) \qquad delta\,(p) = g * \varrho * B,$$

wherein delta (p) stands for the pressure differential (in Pa), $\varrho$ for the density of the liquid (in $kg/m^3$), B for the suction height differential (in m), and g for the gravity constant (in $m/s^2$) can be considered
As the test is executed, the flow rate is determined gravimetrically, and hence the volumetric flow rate Q is replaced by the mass flow rate FR. The connection between mass flow rate FR and volumetric flow rate Q is mass density of the liquid.

Test set up

As shown in Fig. 2, the test stand 1100 comprises a liquid reservoir 1110, sufficiently large to allow minimal height change of the liquid level 1111 (less than 0.1% of the length A+B difference during test execution) of the test fluid 1020, optionally automatic refilling, positioned on a support (e.g. lab jack) 1115, which may be height adjustable (as indicated by direction arrows 1116) and which must be set to height of larger than B (> 200 mm) allowing to adjust B to 200 mm and positioning a balance/scale 1130 below to collect the test liquid.
A liquid receiving receptacle 1120 is positioned on a scale 1130 with an accuracy of ± 0.01 g or better. The receptacle 1120 is positioned below and laterally adjacent close to the support 1115 with the reservoir 1110 with straight sides facing towards each other.

A horizontal support beam 1140, preferably smooth or polished stainless steel rod at diameter sufficient to separate the down hanging strips of test material without kinking, e.g., of 10 mm, is positioned at a siphon height A adjustable to 0.2 mm accuracy (reading at upper diameter of support beam) vertically relative to the liquid level of the reservoir, as is referred to as "siphon height A", read and reported to a millimeter and such that the length of the beam extends over the facing sides of the receptacle and reservoir.

Height measurement devices (not shown) allow determination of

- Siphon height A as the vertical distance of the liquid level 1111 of the reservoir 1110 and the height of the (uppermost height of the) beam, and
- the Suction height B, i.e. the vertical distance between the liquid level in the reservoir 1111 to the lowermost end 1018 of the test material 1010 hanging from the beam 1140 down toward the receptacle 1120.

Further, a suitable timer is required to monitor respective time intervals.

The test liquid 1020 is saline with a concentration of 9 g/l.

Test material preparation

The test material 1010 should be conditioned to laboratory conditions without pressure (e.g., by being unwound to allow zero pressure expansion) and is rectangularly cut to a material width of 40 mm and length of 1000 mm. The length / width orientation of the material should be such that it corresponds to the intended MD/CD positioning in the article.

Care should be taken that the cutting of the test material is executed with a sharp cutting tool (e.g. a scalpel or razor blade), which is not / only minimally impacting on the pore size of the material at the edges by avoiding irreversible compaction of the material.

Test execution

1) 3000 ml of test fluid are filled into the reservoir.

2) The test sample is pre-soaked in test fluid in a separate receptacle outside the reservoir until equilibrium is reached (approximately 10 s).

3) It is then placed over the support beam at a siphon height A of 20 mm with one end hanging freely at a suction height B of 200 mm below the liquid level in the reservoir, such that test fluid is drawn from the reservoir into the receptacle. After two minutes conditioning, the liquid as received in the receptacle may be quickly poured into the reservoir, and the receptacle is placed on the scale again, which is then tared.

4) The reading of the scale is monitored at least every 30 seconds for 2 minutes, allowing to calculate a flow rate $FR(A=20)$, expressed in $g_{liq}/min$, for this setting.

Then, the siphon height A(i) is increased in regular intervals of 20 mm until a maximum siphon height A of 200 mm is reached, and the respective flow rates FR (A=i) up to FR (A=200), expressed in $g_{liq}/min$, are determined.

The corresponding siphon flow length, i.e. the path the liquid flows from the reservoir to the end of the material, corresponds to

$$(Eq\text{-}3) \qquad L = 2*A + B \, .$$

After each setting, the liquid level in the reservoir is readjusted, either by adding fresh test fluid, or reusing test fluid from same day runs from the receptacle, if the samples do not contain compounds that may leach out impacting the surface tension of the test liquid. Each three replicates are run with fresh material stripes, and the results are averaged.

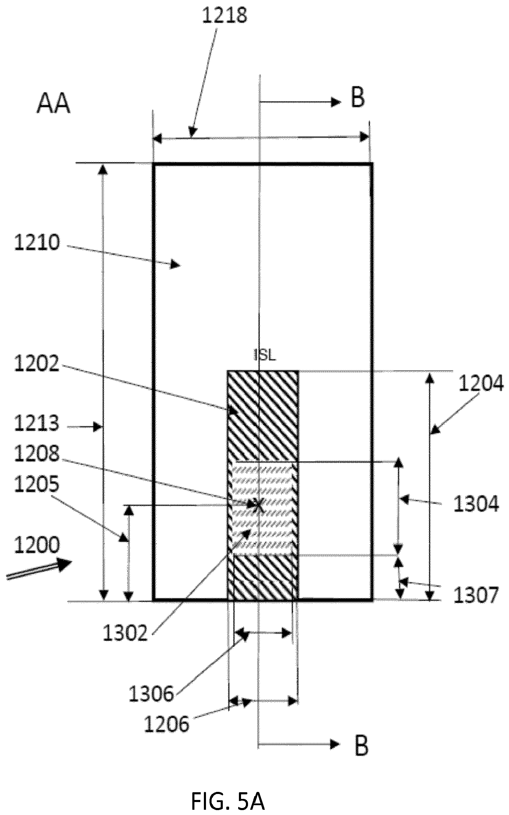

A typical result is depicted in Fig. 5A, showing the points of the measurements 5110 of the flow rates FR as a function of the total length L, as well as a well matching exponential fit curve 5120.

Test results

Stall height

Without wishing to be bound by the theory as laid out in Hagen-Poiseuille and Laplace equations, it is believed that an important aspect for the liquid transport in the present system is the siphon height or "stall height" ("SH") which refers to the suction height A(SH) where capillary and suction forces are balanced and air is sucked into porous matrix, thereby reducing the flow rate by blocking pores with air becoming impermeable for liquid. To determine the specific siphon or stall height A, the measured flow rate is compared to the theoretical flow rate, which would occur if the test material would behave like a closed tube and would follow the law of Hagen-Poiseuille, as is exemplarily explained in the context of Fig. 5A to 5D.

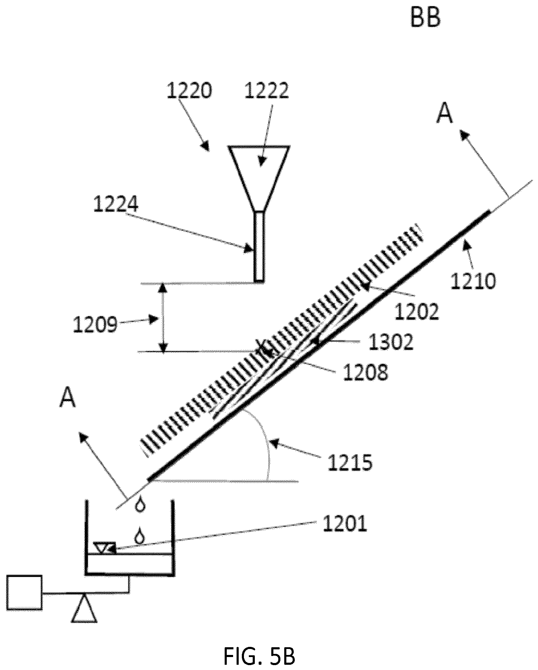

If the test strip behaves like a theoretically closed tube, plotting the inverse flow rates 1/FR against the tube length L, a linear function of type 1/FR = a* L + b would result according to the law of Hagen-Poiseuille - see theoretical points 5230 in Fig. 5B. However, as also depicted in Fig. 5B, when plotting the experimentally obtained results 5210 of the inverse of the flow rates 1/FR versus the liquid flow path L, it shows again an exponential function 5220, because air is sucked through the pores when increasing the siphon height above the wicking height, which decreases the flow rate additionally to the increase of the flow resistance with longer tube lengths. To determine the (stall) height, at which the test stripe does not behave like a closed tube anymore, the point of fading linearity of the exponential function was determined. Therefore, as depicted in Fig 5C, several linear regressions at the previously obtained exponential regression were conducted, including increasing number of points, always starting at the lowest siphon height (L = 0.24 m, with A = 20 mm and B = 200 mm, 5300) and the next point 5301, resulting in a regression coefficient $R^2$ of 1.000, see regression line 5310 and consecutively including an increasing number of data points, i.e. the second regression line 5311 for points 5300, 5301, and 5302, resulting in corresponding correlation coefficients, the third regression line 5312 for points 5300, 5301, 5302, 5304 and so on (the last lines being omitted for clarity). Referring to Fig. 5D, the corresponding regression coefficients ($R^2$, y-axis) were plotted against the tube length (i.e. L = (2*A+B), x-axis), which typically resulted again in a second order polynomial function, of which the coefficient $R^{2'}$ were determined.

In a next step, the regression coefficients 5401 to 5410 are plotted versus the total length L, see Fig. 5D. It is considered, that a linear correlation exists for a correlation coefficient of not less than 0.9800, whilst below this value the correlation is not linear anymore, and thus the stall height length at point 5430, e.g. in Fig. 5D of $L_{SH}$ of about 0.42 m, that corresponds to a stall height SH of about 110 mm.

The mathematically derived stall heights are in good agreement with the visual observation that the test stripes get dried out starting on the top of the siphon at a height, which correlates with the stall height.

It might be noted, that various parameter of the material may be summarized in the matter constant K which also can be derived from the experimentally obtained flow rates, which were plotted against the quotient of suction height B and the entire length of the test stripe being outside the reservoir (2+B). The resulting curve is linear over a wide range, so that a linear regression including x-values up to the previously determined stall height (SH) of the specific material gives good correlations with correlation coefficients above 0.98 and even higher. The slope of the linear regression corresponds to the matter constant K of the nonwoven test stripe, which, when rearranging

$$(Eq\text{-}1) \qquad Q \text{ - } FR = (r^4 * \pi * \text{delta } (p))/ (8* \eta *L),$$

with

$$(Eq\text{-}2) \qquad \text{delta } (p) = g*\varrho*B,$$

towards

$$(Eq\text{-}4a) \qquad Q = K * B/L$$

wherein the matter constant K is

$$(Eq\text{-}4b) \qquad K = (r^4 * \pi * g*\varrho)/ (8* \eta).$$

Dynamic ISM Material Run-off

This test aims at assessing the ability of a material, in particular a material suitable as ISM material, to receive, distribute and retain a test liquid, which is deposited under gravity driven flow conditions onto the material at an inclined position.

The test methods is a modification of the known NWSP Test method NWSP 080.9.R0 (15) "Nonwoven run-off".

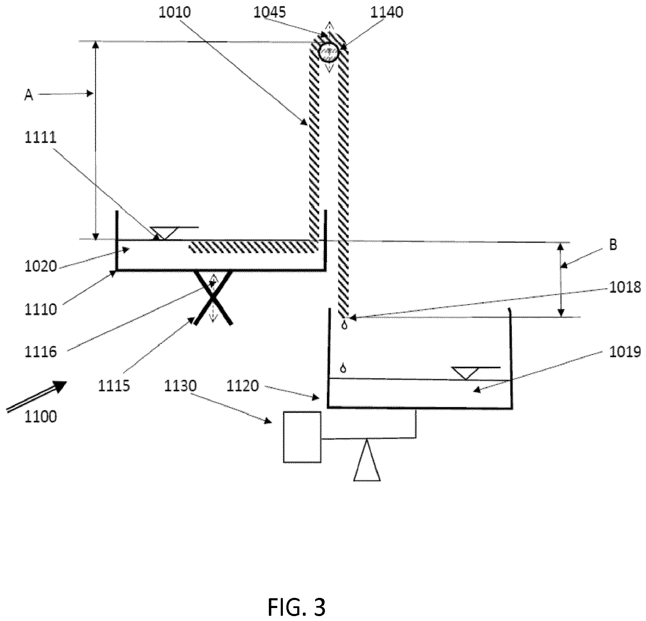

Test equipment as shown in Fig. 3A and B is based on the NWSP 080.9, comprising the following:

- A Run-off table 1210 made of acrylic glass or similar exhibiting a size of at least the size of the samples, e.g. having a length 1212 of about 645 mm and a width 1218 of about 320 mm at an incline at an angle 1215 of 25° +/- 0.17°, optionally comprising marker lines for positioning the test specimen and fixation means, such as a clip or adhesive fixation tape. The table should not be tilted and be horizontally well aligned in the width direction.

- a tube 1224 with an internal diameter of 5 mm is adapted to deliver the test liquid at a fixed position and height, e.g. by means of a ring stand.

  - a liquid dosing means 1220 adapted to delivering the predetermined amount of test liquid in a continuous stream via the tube within a predetermined liquid delivery time, e.g. either a funnel 1222 with a valve to regulate the average liquid flow, or a syringe with a motorized syringe drive unit, or a hydraulic pump or another pressurized system, leak free attached to the tube.

- a timer, which is capable of measuring 60 s to an accuracy of 0.1 s, and
- an analytical balance, which is capable of determining a mass of at least 50 g to an accuracy of 0.01g.

In contrast to the NWSP method, the standard absorption medium, which is placed below the sample and test pad for receiving run-off liquid is omitted and replaced by a well-tared liquid receptacle of a size to receive liquid as may run-off the plate.

As test fluid 1201, saline with a concentration of 9 g/l is employed.

Sample preparation

A test specimen 1202 is prepared by cutting the material to a length 1204 of 300 mm and a width 1206 of 100 mm, whereby the length direction is aligned with the machine direction of the article when making and also with the length direction of the article, once used.

Care should be taken that at cutting the material is not unduly compressed, such by using a sharply cutting cutter.

If the test specimen exhibits surfaces differing in properties the appropriate surface shall be determined and noted. Unless differently noted, the surface, which is intended to be oriented towards the skin under in-use conditions, is turned to upwardly in the test rig to receive the liquid.

Test preparation

The conditioned and pre-weighed test specimen is affixed to the run-off table 1210 such that the width direction 1206 is aligned with the width of the table 1218 and the lower end of the specimen is positioned until flush with the lower end of te the table 1210.

The lower end of the tube 1224 for delivering the test liquid is positioned vertically at a liquid load height 1209 of 25 mm $\pm$ 1 mm above the liquid loading point 1208 of the material, which is at a distance 1205 of 125 mm $\pm$ 1 mm upwardly of the lower end of the test specimen.

The liquid delivery device is adjusted to release liquid at a rate of 50 g +/- 0.2 g of saline in 3.8 +/- 0.1 sec.

Test execution

The conditioned test specimen is weighed and the mass noted as m (dry), before it is positioned on the inclined run-off table. 50 g of saline are released through the tube 1224 at the predetermined and pre-set rate, and the timer is started when the liquid hits the test specimen to. After 5 min, the test specimen is carefully transferred to the scale and the mass of the wet specimen is noted m (wet) as well as the amount of drip-off, if present, as accumulated in the receptacle.

Reporting Test results

The amount of liquid in the wet specimen is referred to as *intermediate storage capacity* and may be expressed in $g_{liq}/g_{mat}$ for the ISM materials (grams liquid per grams material) or percentage (weight-% of liquid load). If the mass increase of the specimen and the amount of drip-off do not add to the liquid load within 97 %, the test results should be discarded.

Static ISM Material Run-off

This test aims at assessing the storage capacity of the ISM material if a specimen is fully saturated with the test liquid and affixed to the inclined run-off table. The test is executed as the above described Dynamic ISM Material Run-off test, with the following modifications:
The test specimen is soaked in test liquid for 1 min and subsequently transferred to a horizontal steel grid of a mesh size of 10 mm, where the liquid can drip out from the specimen without any pressure applied for 1 min to be then transferred carefully without squeezing, that might induce liquid loss, to the inclined run-off table 1210 as of the Dynamic ISM Material Run-off test. After 5 min waiting period, the amount of run-off liquid is recorded and the ISM material is carefully transferred to the scale and re-weighed. The resulting data may be expressed in absorbance capacity in $g_{liq}/g_{mat}$ for the ISM materials or percentage (weight-% of liquid load) for ISM and leakage.

Combined ISM and USM Material Run-off test

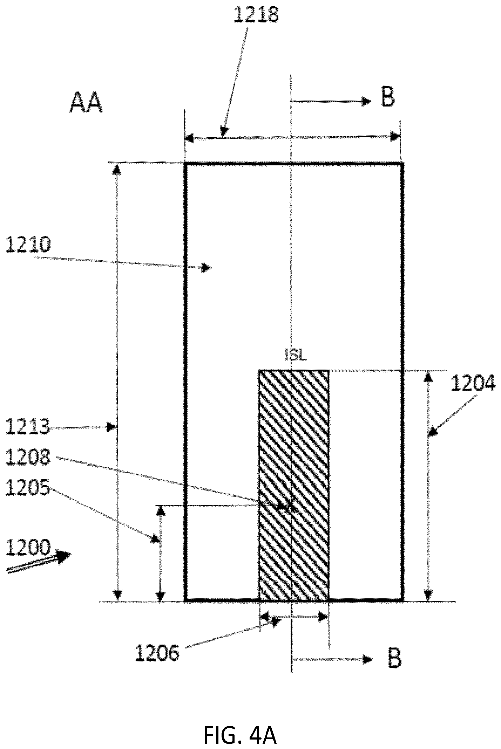

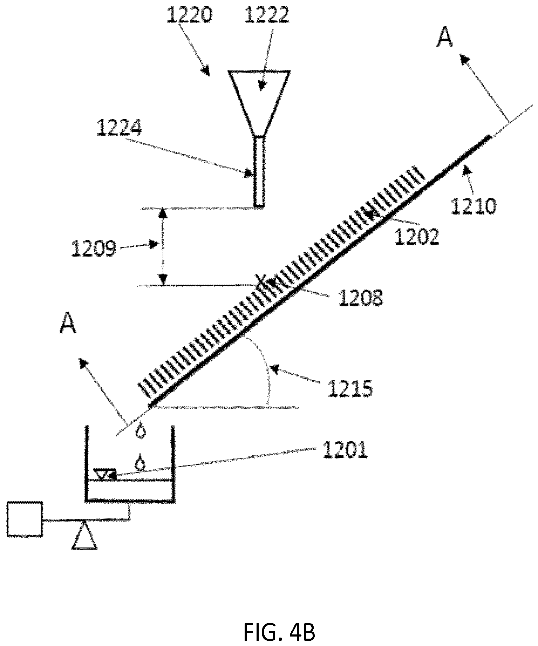

The test aims at assessing the interaction of the ISM and the USM materials by evaluating the partitioning or distribution of liquid within a combination of ISM and USM. The test equipment and set-up and execution (see Fig. 4) is essentially the same as described in the context of Fig. 3, except for positioning a patch of USM material 1302 between the ISM material 1202 and the test plate 1210. Unless expressly stated, the USM material patch has a size of 100 mm length and 100 mm width and is conditioned and prepared in analogy to the ISM material. For both materials the respective dry weight m (dry) are determined and recorded. The position of the USM material patch longitudinal aligned with the centre line of the ISM material. The longitudinal centre point of the patch is placed in registry with the centre point of the ISM and the lower edge is positioned at an USM edge to ISM edge distance 1307 of 100 mm from the lower end of the ISM material and thus the lower end of the run-off table 1210. After a waiting period of 10 mins after application of the liquid load as described in the above, the amount of run-off liquid is recorded from the scale and both the ISM material and the USM patch are carefully transferred to the scale and weights are noted as m(wet). All acquired run-off data may be expressed in absorbance capacity in $g_{liq}/g_{mat}$ for the ISM and USM materials or percentage (weight-% of liquid load) for ISM and USM materials as well as run-off leakage.

In the first variant, the test is executed with a liquid load of 50 $g_{liq}$ for all test material combinations.

In a second variant the test is executed with an adjusted amount of test liquid load according to the basis weight of the ISM material, referenced to a material with 120 $g_{mat}/m^2$ basis weight (e.g. example ISM-3) with 50 g liquid load.

In a third variant, the test is executed with an adjusted amount of test liquid load according to the intermediate storage capacity (ISC) determined by the method "Dynamic ISM Material Run-Off" (as indicated for the examples in Table3), whereby for a material exhibiting an ISC of less than 9 $g_{liq}/g_{mat}$, the load is set to 10 $g_{liq}$, for ISC values from 9 $g_{liq}/g_{mat}$ to 11 $g_{liq}$ the load is set to 25 ml, and for ISC values of more than 11 $g_{liq}/g_{mat}$ the load is set to 40 $g_{liq}$.

ISM recovery test

This test aims at demonstrating the capability of the ISM and USM to enable the ISM to recover after a liquid load by moving liquid into the USM so as to be ready for subsequent gushes. This test is based on the "Dynamic ISM/USM Liquid Distribution Test" as described hereinabove. After application of the test liquid on the assembly of ISM and USM patches and waiting for 10 minutes giving the USM material time to suck liquid out of the ISM into the USM, the distribution of the applied test liquid between ISM material and USM material as well as the amount of leaked out liquid is determined. Then the USM patch is carefully replaced by a fresh USM patch whilst avoiding than any liquid from the ISM material is lost. Then, the liquid loading is repeated using the same ISM without removing the included liquid from the previous gush. After a further waiting period of 10 minutes, the distribution of the applied test liquid and the leaked out liquid get determined by measuring the weight of ISM, USM and leaked off liquid. This is repeated a third time and after a final waiting period of 10 minutes the rewet of the ISM/USM assembly is measured NWSP 070.9.R1 (15). The filter paper is pressed onto the ISM under load of 2 x 4 kg. The reported data are the liquid distribution for each gush in % of the load, or the respective liquid loads as well as the rewet value in g.

"Repeated ISM/USM Post Acquisition Rewet Test"

This test aims at assessing the acquisition time and rewet in a horizontal position of an absorbent system by combining an ISM patch of 300 mm x 100 mm with a USM patch of 100 mm x 100 mm with the USM centred under the ISM at the application point of the liquid. The test is performed according to NWSP 070.8.R0 (15) with the modification of replacing the 10 plies of filter paper by the absorbent system and by having a 10 minutes wait period between the gushes. The liquid load was set to 5 ml per gush. The rewet is determined after the 3rd gush and a waiting period of 10 minutes. In case of liquid seeping out at the sides (rather than entering into the material), the sample may be enveloped in a plastic film, e.g. typical backsheet film for absorbent articles, without covering the area over which liquid is applied.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

**Claims**

1.  An absorbent hygiene article having an x- or length direction, a y- or width direction and a z- or thickness direction, said absorbent hygiene article comprising:

    a topsheet;
    a backsheet; and
    a liquid absorbent system disposed therebetween, the liquid absorbent system comprising:

    A) an ultimate liquid storage region,
    comprising an Ultimate Storage Member (USM) wherein said USM

    - comprises
    A1) USM material exhibiting a basis absorbent retention of at least 2000 ml/m$^2$, whereby said USM material comprises SAP material;
    - and exhibits
    A2) a dry thickness of less than about 3.0 mm, preferably less than about 1.0 mm, more preferably of less than about 0.8 mm, and
    B) an intermediate storage region comprising an Intermediate Storage Member (ISM) comprising an ISM material, said ISM material exhibiting
    B1) in the "Siphoning test"

    B1a) - a Capillary Stall Height of more than about 45 mm, preferably of more than about 60 mm, even more preferably more than about 80 mm;
    B2) - in the "Dynamic ISM Run-off Test"
    B2) an Intermediate Storage Capacity of at least about 6.7 $g_{liq}$/g, preferably of at least about 9.1 $g_{liq}$/g, more preferably more than about 11.0 $g_{liq}$/g,

    wherein in said liquid absorbent system

    C1) said ultimate liquid storage region exhibits x-y-directionally an area of less than 85%, preferably less than 75% more preferably less than about 50% of the area of the intermediate storage region, and
    C2) said USM exhibits a capacity of at least about 2500 g/m$^2$, preferably at least about 3000 g/m$^2$, more preferably at least about 4000 g/m$^2$, when submitted to the "Combined ISM and USM Material Run-off Test" upon application of a liquid load adapted to the basis weight of the overlying ISM" .

2.  An absorbent hygiene article as claimed in claim 1, wherein said USM material further satisfies one or more of the conditions selected from the group consisting of

    A3) exhibiting a flexural rigidity (x-directional) of less than about 35 mN*cm, preferably less than 20 mN*cm, more preferably less than 5 mN*cm;
    A4) exhibiting a Circular Bending of less than about 3.8 N, preferably less than about 2.7 N;
    A5) exhibiting a compressibility of less than 9 %;
    A6) exhibiting an absorbent retention capacity of more than about 15 $g_{liq}$/g, preferably more than 20 $g_{liq}$/g. even more preferably more than 25 $g_{liq}$/g and most preferably of more than 30 $g_{liq}$/g;
    A7) exhibiting an absorbent retention Basis Capacity of at least about 25 $g_{liq}$/m$^2$, preferably at least about 28 $g_{liq}$/m$^2$, more preferably at least about 40 $g_{liq}$/m$^2$;
    A8) an absorbent retention effective capacity of more than about 3 $g_{liq}$/cm$^3$.

3. An absorbent hygiene article as claimed in claim 1 or claim 2, wherein said ISM material further satisfies one or more of the conditions selected from the group consisting of

  B1) exhibiting in the "Siphoning test"

    B1b) - a Flow Rate of at least 1.7 $g_{liq}$/ min, preferably more than about 2.8 $g_{liq}$/ min, even more preferably of more than 4.1 $g_{liq}$/ min
    B1c) - a vertical wicking of more than 35 mm, preferably more than 65 mm, most preferably more than 90 mm;
    B2) - in the "Dynamic ISM Run-off Test"
    B2b) a run-off value of less than 50%, preferably less than 20% and most preferably of essentially zero %;

  B3) exhibiting in the "static ISM run-off test";

    B3a) an absorption capacity of at least about 14 g/g, preferably of at least about 15 g/g, more preferably more than about 18 g/g;
    B3b) a Run-off of less than about 40%, preferably less than about 20%, more preferably less than about 15%;

  B4) exhibiting a flexural rigidity (MD) of less than 32 mN*cm, preferably less than 27 mN*cm, more preferably less than 5 mN*cm;
  B5) exhibiting a Circular Bending of less than about 0.75 N, preferably less than about 0.50 N, more preferably less than 0.40 N;
  B6) a compressibility of more than about 14 %, preferably more than about 15 %, more preferably more than about 18%.

4. An absorbent hygiene article according to any of the preceding claims, wherein the combination of said ISM material and said USM material satisfies at least one of the requirements selected from the group consisting of

  C3) exhibiting, when tested according to the 1st variant of the "Dynamic ISM/USM Liquid Distribution Test" with a liquid load of a fixed amount of test fluid of 50 g,

    C3a) a USM loading of at least about 40%, preferably more than about 54 %, and even more preferably at least about 80% of the test fluid;
    C3b) a run-off of not more than about 60%, preferably less than about 30%, even more preferably less than about 10%, or even no detectable run-off at all;

  C4) exhibiting, when tested according to the 2nd "variant of the "Dynamic ISM/USM Liquid Distribution Test", after loading with an amount of test fluid being adapted to the intermediate storage capacity of the ISM as determined according to the "Dynamic ISM run-off test",

    C4a) a USM loading of more than about 60 %, preferably more than 75 %, and even more preferably more than about 78 % of the liquid load;
    C4b) a Run-off of less than about 30%, preferably less than about 5 %, and most preferably essentially zero % run-off;

  C5) exhibiting when tested according to the 3rd "variant of the "Dynamic ISM/USM Liquid Distribution Test"," after loading with an amount of test fluid being adapted to the basis weight of the ISM,

    C5a) a USM loading of more than about 60 %, preferably more than 75 %, and even more preferably more than about 78 % of the liquid load;
    C5b) a run-off of less than about 30%, preferably less than about 5 %, and most preferably essentially zero % run-off;

  C6) exhibiting when tested according to the "Repeated ISM/USM Post Acquisition Rewet Test" exhibiting a rewet value of less than about 0.15 g, preferably less than about 0.05 g rewet;
  C7) (Table 10) exhibiting when tested according to the "ISM recovery test at multiple loading
  C7a) a difference of less than 30%, preferably less than 10% between the 2nd and 3rd loading for

    C7ai) the ISM load in % of the load;

C7aii) the ISM load in $g_{liq}/g_{mat}$;
C7iii) ISM load in $g_{liq}/m^2$;

C8) the flexural rigidity of said ISM being lower than the flexural rigidity of the USM; C9) the Circular Bending of said ISM being lower than the Circular bending of the USM.

5. An absorbent hygiene article according to any of the preceding claims, wherein said ISM material comprises synthetic fibers, preferably bicomponent fibers, and cellulosic, preferably pulp, fibers, and a latex binder.

6. An absorbent hygiene article according to claim 5, wherein said ISM material is of the airlaid material type.

7. An absorbent hygiene article according to any of the preceding claims, wherein said USM material comprises SAP material at a concentration of at least about 80%, preferably at least about 90 % and more preferably of at least 95%, and further comprises cellulose, preferably pulp, and synthetic fibers.

8. An absorbent hygiene article according claim 7, wherein said USM material further comprises latex and is of the airlaid material type.

9. An absorbent hygiene article as claimed in any preceding claim for positioning in the lower waist region of a wearer and said article comprising in Cartesian coordinates

a length or longitudinal extension or x-direction,
a width extension
and a thickness extension perpendicular to the length and the width;

said article comprising

a front portion, for being positioned towards the front waist region of a wearer during use;
a rear portion, for being positioned towards the rear waist region of a wearer during use;
and a crotch portion positioned between the front and the rear portion;
a first surface, intended

to be oriented towards a wearer during use, also referred to as topsheet surface and adapted to receive liquid bodily exudates of said wearer,
preferably the surface of an article cover or topsheet;

a z-directional opposite surface, intended to be oriented outwardly and away from a wearer;

wherein said article further comprises an absorbent system according to any of the preceding claims positioned between said first and opposite surface of the article and aligned with the corresponding x-, y-, and z-direction of said article,
such that said ISM region is positioned towards said first surface, and said USM region is positioned towards said opposite surface.

10. An absorbent hygiene article according to claim 9, wherein said article is further selected from the group consisting of:

D1) a day use feminine hygiene article exhibiting an article retention capacity of more than about 2 ml but less than about 7 ml;
D2) a medium capacity hygiene pads exhibiting an article retention capacity of more than about 10 ml but less than about 50 ml;
D3) a high capacity incontinence article exhibiting an article retention capacity of more than about 50 ml, but less than about 300 ml;
D4) a ultra high capacity incontinence article exhibiting an article retention capacity of more than about 300 ml,

wherein said articles comprise

- a USM material exhibiting a retention capacity of more than about 10 $g_{liq}/g_{mat}$, preferably more than 15 $g_{liq}/g_{mat}$, more preferably more than 19 $g_{liq}/g_{mat}$, and an ISM exhibiting an ISM intermediate absorption capacity of at least about 5 $g_{liq}/g_{mat}$, preferably at least about 6 $g_{liq}/g_{mat}$, more preferably of at least about 9 $g_{liq}/g_{mat}$, and even more preferably of at least about 11 $g_{liq}/g_{mat}$.

FIG. 1

FIG. 2

FIG. 3

FIG. 4A

FIG. 4B

FIG. 5A

FIG. 5B

FIG. 6A

FIG. 6B

FIG. 6C

FIG. 6D

Europäisches
Patentamt

European
Patent Office

Office européen
des brevets

# EUROPEAN SEARCH REPORT

**Application Number**

EP 18 17 2397

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/069964 A1 (SCA HYGIENE PROD AB [SE]; YAZARLO SHADI [SE]; ANDERSSON PATRIK [SE]; H) 21 June 2007 (2007-06-21) | 1-4,7-10 | INV. A61F13/537 |
| A | * figure 3 * <br> * p. 3, second full paragraph; p. 9, last paragraph; Examples * <br> * table 1 * <br> * p. 9, last paragraph; p. 16, last paragraph; * <br> ----- | 5,6 | |
| A | WO 2016/040097 A1 (PROCTER & GAMBLE [US]) 17 March 2016 (2016-03-17) <br> * figures 2,12A, 14 * <br> ----- | 1-10 | |
| A,D | WO 2014/009506 A1 (GLATFELTER FALKENHAGEN GMBH [DE]) 16 January 2014 (2014-01-16) <br> * the whole document * <br> ----- | 1-10 | |

**TECHNICAL FIELDS SEARCHED (IPC)**

A61F

The present search report has been drawn up for all claims

| Place of search | Date of completion of the search | Examiner |
|---|---|---|
| Munich | 18 October 2018 | Mauhin, Viviane |

EPO FORM 1503 03.82 (P04C01)

## ANNEX TO THE EUROPEAN SEARCH REPORT
## ON EUROPEAN PATENT APPLICATION NO.

EP 18 17 2397

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2018

| Patent document cited in search report | | Publication date | Patent family member(s) | | Publication date |
|---|---|---|---|---|---|
| WO 2007069964 | A1 | 21-06-2007 | AR | 058489 A1 | 06-02-2008 |
| | | | AU | 2005339199 A1 | 21-06-2007 |
| | | | BR | PI0520752 A2 | 26-05-2009 |
| | | | CA | 2630855 A1 | 21-06-2007 |
| | | | CN | 101325939 A | 17-12-2008 |
| | | | EP | 1959905 A1 | 27-08-2008 |
| | | | JP | 2009519098 A | 14-05-2009 |
| | | | TN | SN08211 A1 | 30-10-2009 |
| | | | TW | 200727873 A | 01-08-2007 |
| | | | US | 2011106036 A1 | 05-05-2011 |
| | | | WO | 2007069964 A1 | 21-06-2007 |
| WO 2016040097 | A1 | 17-03-2016 | CN | 106687088 A | 17-05-2017 |
| | | | EP | 3191047 A1 | 19-07-2017 |
| | | | JP | 2017530765 A | 19-10-2017 |
| | | | WO | 2016040097 A1 | 17-03-2016 |
| WO 2014009506 | A1 | 16-01-2014 | AU | 2013288682 A1 | 05-02-2015 |
| | | | BR | 112015000652 A2 | 27-06-2017 |
| | | | CA | 2878794 A1 | 16-01-2014 |
| | | | CN | 104540488 A | 22-04-2015 |
| | | | EP | 2872097 A1 | 20-05-2015 |
| | | | GB | 2503924 A | 15-01-2014 |
| | | | JP | 6272852 B2 | 31-01-2018 |
| | | | JP | 2015525628 A | 07-09-2015 |
| | | | KR | 20150031475 A | 24-03-2015 |
| | | | RU | 2015104865 A | 27-08-2016 |
| | | | US | 2015164710 A1 | 18-06-2015 |
| | | | WO | 2014009506 A1 | 16-01-2014 |

EPO FORM P0459

For more details about this annex : see Official Journal of the European Patent Office, No. 12/82

## REFERENCES CITED IN THE DESCRIPTION

**Patent documents cited in the description**

- US 4699619 A
- EP 1075241 A
- EP 1061878 A
- US 6506960 B
- US 6727403 B
- WO 2000000138 A1
- EP 1091714 A2

- EP 1061878 A1
- EP 10611878 A
- EP 2872097 A
- WO 03090656 A1
- US 2002007169 A1
- WO 0074620 A1
- WO 2011084981 A1

**Non-patent literature cited in the description**

- **H. RÖTTGER.** Ultrathin Absorbent Solutions Enabling Design of Garment Like Disposable. *Insight Conference,* 2014